(19) Europäisches Patentamt / European Patent Office / Office européen des brevets

(11) **EP 4 682 522 A1**

(12) **EUROPEAN PATENT APPLICATION**
published in accordance with Art. 153(4) EPC

(43) Date of publication:
**21.01.2026 Bulletin 2026/04**

(21) Application number: **24770294.7**

(22) Date of filing: **06.02.2024**

(51) International Patent Classification (IPC):
**G01N 25/56** (2006.01)    **G01N 29/024** (2006.01)

(52) Cooperative Patent Classification (CPC):
**G01N 25/56; G01N 29/024**

(86) International application number:
**PCT/JP2024/003808**

(87) International publication number:
**WO 2024/190174 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.03.2023 JP 2023039802**

(71) Applicant: **PANASONIC INTELLECTUAL PROPERTY MANAGEMENT CO., LTD.**
**Kadoma-shi, Osaka 571-0057 (JP)**

(72) Inventors:
• **TAKAKURA, Yuya**
**Osaka 571-0057 (JP)**

• **MATSUDA, Masataka**
**Osaka 571-0057 (JP)**
• **NAKABAYASHI, Yuuji**
**Osaka 571-0057 (JP)**
• **SATOU, Masato**
**Osaka 571-0057 (JP)**
• **KONISHI, Ryohei**
**Osaka 571-0057 (JP)**
• **MIYOSHI, Asako**
**Osaka 571-0057 (JP)**

(74) Representative: **SSM Sandmair**
**Patentanwälte Rechtsanwalt**
**Partnerschaft mbB**
**Joseph-Wild-Straße 20**
**81829 München (DE)**

(54) **PHYSICAL QUANTITY MEASUREMENT SYSTEM**

(57) The problem to be overcome by the present disclosure is to more accurately measure the water vapor pressure of gas mixtures even when the gas mixtures flow through a flow channel under a high-humidity environment. A physical quantity measurement system (1) includes a temperature and humidity sensor (16), a heat generator (17), a heat generation controller (203), and a water vapor pressure measurer (204). The heat generator (17) performs a heat generating operation that generates heat to warm the temperature and humidity sensor (16). The water vapor pressure measurer (204) measures a water vapor pressure of water vapor included in the gas mixtures. The heat generation controller (203) controls ON/OFF states of the heat generator (17) based on a result of comparison between a humidity measured value measured by the temperature and humidity sensor (16) and a threshold value. The water vapor pressure measurer (204) measures, irrespective of the state of the heat generator (17), the water vapor pressure of the water vapor based on the humidity measured value and a temperature measured value measured by the temperature and humidity sensor (16).

EP 4 682 522 A1

# FIG. 1

## Description

### Technical Field

The present disclosure generally relates to a physical quantity measurement system, and more particularly relates to a physical quantity measurement system, of which the target of measurement is gas mixtures flowing through a flow channel.

### Background Art

A fuel cell system, for example, has been known in the art as a system which uses a physical quantity measurement system for measuring a hydrogen concentration in a flow channel through which a mixed gas (gas mixtures) including hydrogen flows (see, for example, Patent Literature 1).

The fuel cell system of Patent Literature 1 teaches that when a mixed gas including three components, namely, hydrogen, water vapor, and an impurity gas, flows through a flow channel, a hygrometer is provided to obtain a water vapor concentration using the result of detection by the hygrometer.

According to a known method, when a water vapor concentration is obtained from gas mixtures including three components, namely, hydrogen, water vapor, and an impurity gas, first, the water vapor pressure is measured by using the result of detection (humidity) measured by a hygrometer. If a system for measuring a physical quantity (such as a water vapor concentration) (i.e., a physical quantity measurement system) is used under a high-humidity environment, condensation may be produced in the hygrometer. If any condensation is produced, then the accuracy of humidity measurement using the hygrometer will decline, thus causing a decrease in the accuracy of measurement of the water vapor pressure, which is a problem with the known method.

### Citation List

### Patent Literature

Patent Literature 1: JP 2003-317752 A

### Summary of Invention

In view of the foregoing background, it is therefore an object of the present disclosure to provide a physical quantity measurement system having the ability to more accurately measure the water vapor pressure of gas mixtures even when the gas mixtures flow through a flow channel under a high-humidity environment.

A physical quantity measurement system according to an aspect of the present disclosure includes a temperature and humidity sensor, a heat generator, a heat generation controller, and a water vapor pressure measurer. The temperature and humidity sensor measures a humidity and temperature of gas mixtures flowing through a flow channel. The heat generator performs a heat generating operation that generates heat to warm the temperature and humidity sensor. The heat generation controller controls ON/OFF states of the heat generator to cause the heat generator to switch from an ON state where the heat generator performs the heat generating operation to an OFF state where the heat generator stops performing the heat generating operation, and vice versa. The water vapor pressure measurer measures a water vapor pressure of water vapor included in the gas mixtures. The heat generation controller controls the ON/OFF states of the heat generator based on a result of comparison between a humidity measured value of the gas mixtures that has been measured by the temperature and humidity sensor and a threshold value. The water vapor pressure measurer measures, irrespective of a state of the heat generator, the water vapor pressure of the water vapor included in the gas mixtures based on the humidity measured value and a temperature measured value of the gas mixtures that has been measured by the temperature and humidity sensor.

### Brief Description of Drawings

[FIG. 1] FIG. 1 is a block diagram illustrating a configuration for a physical quantity measurement system according to an exemplary embodiment;
[FIG. 2] FIG. 2 is a system diagram illustrating a configuration for the physical quantity measurement system;
[FIG. 3] FIG. 3 is a cross-sectional view, taken along an X-Z plane, of a flow channel body included in the physical quantity measurement system;

[FIG. 4] FIG. 4 is a cross-sectional view, taken along an X-Y plane, of the flow channel body included in the physical quantity measurement system;

[FIG. 5] FIG. 5 is a flowchart showing the procedure of operation of the physical quantity measurement system;

[FIG. 6] FIG. 6 is a flowchart showing the procedure of heat generation control processing to be performed by the physical quantity measurement system;

[FIG. 7] FIG. 7 is a flowchart showing the procedure of third measurement processing to be performed by the physical quantity measurement system;

[FIG. 8] FIG. 8 is a flowchart showing the procedure of first abnormality detection processing to be performed by the physical quantity measurement system;

[FIG. 9] FIG. 9 is a flowchart showing the procedure of second abnormality detection processing to be performed by the physical quantity measurement system;

[FIG. 10] FIG. 10 is a flowchart showing the procedure of concentration measurement processing to be performed by the physical quantity measurement system;

[FIG. 11] FIG. 11 is a flowchart showing the procedure of speed of sound calculation processing to be performed as a part of the concentration measurement processing;

[FIG. 12] FIG. 12 is a flowchart showing the procedure of hydrogen concentration calculation processing to be performed as a part of the concentration measurement processing;

[FIG. 13] FIG. 13 is a flowchart showing the procedure of flow rate measurement processing to be performed by the physical quantity measurement system;

[FIG. 14] FIG. 14 is a flowchart showing the procedure of flow coefficient calculation processing to be performed as a part of the flow rate measurement processing;

[FIG. 15] FIG. 15 is a flowchart showing the procedure of relative humidity measurement processing to be performed by the physical quantity measurement system; and

[FIG. 16] FIG. 16 is a flowchart showing the procedure of third measurement processing according to a first variation.

**Description of Embodiments**

Note that the embodiment and its variations to be described below are only an exemplary one of various embodiments of the present disclosure and its variations and should not be construed as limiting the scope of the present disclosure. Rather, the exemplary embodiment and its variations may be readily modified in various manners depending on a design choice or any other factor without departing from a true spirit and scope of the present disclosure.

(Embodiment)

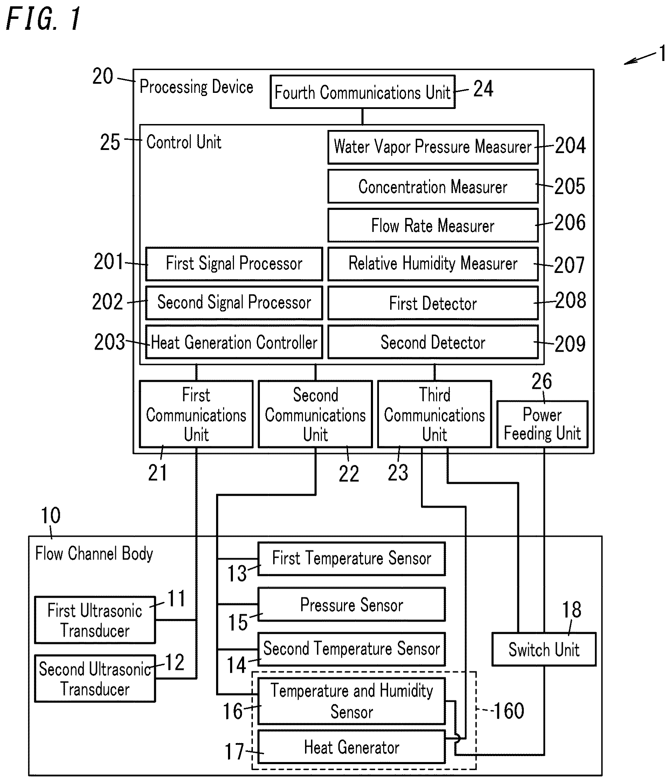

A physical quantity measurement system 1 according to this embodiment will be described with reference to FIGS. 1-15.

(1) Overview

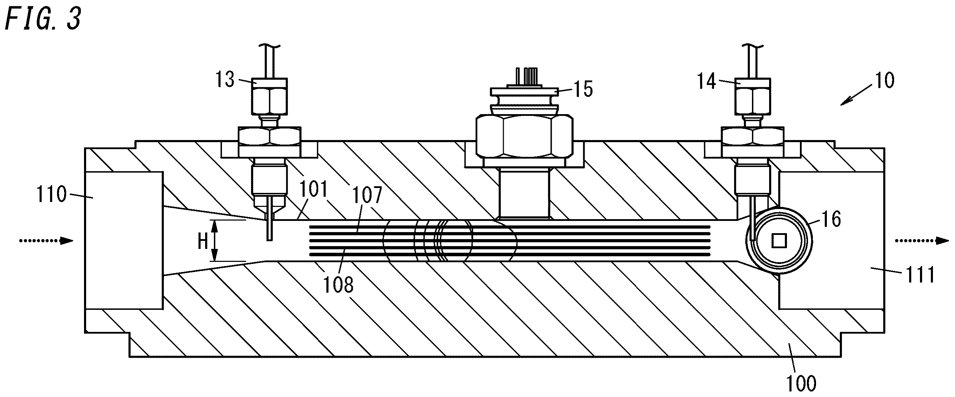

A physical quantity measurement system 1 according to this embodiment is a system for measuring, in gas mixtures including multiple types of gases flowing through a flow channel 101 (refer to FIG. 3), at least one of the water vapor pressure of water vapor as a specific gas, or the relative humidity of the gas mixtures. For example, gas mixtures including hydrogen, nitrogen, and water vapor flow through the flow channel 101.

A physical quantity measurement system 1 according to this embodiment includes a temperature and humidity sensor 16, a heat generator 17, a heat generation controller 203, and a water vapor pressure measurer 204 as shown in FIG. 1. The temperature and humidity sensor 16 measures a humidity and temperature of gas mixtures flowing through a flow channel 101. The heat generator 17 performs a heat generating operation that generates heat to warm the temperature and humidity sensor 16. The heat generation controller 203 controls ON/OFF states of the heat generator 17 to cause the heat generator 17 to switch from an ON state where the heat generator 17 performs the heat generating operation to an OFF state where the heat generator 17 stops performing the heat generating operation, and vice versa. The heat generation controller 203 controls the ON/OFF states of the heat generator 17 based on a result of comparison between a humidity measured value of the gas mixtures that has been measured by the temperature and humidity sensor 16 and a threshold value. The water vapor pressure measurer 204 measures a water vapor pressure of water vapor included in the gas mixtures. The water vapor pressure measurer 204 measures, irrespective of the state of the heat generator 17, the water vapor pressure of the water vapor based on the humidity measured value and a temperature measured value of the gas mixtures that has been measured by the temperature and humidity sensor 16.

This configuration may reduce, by controlling the heat generation operation by the heat generator 17, the likelihood of producing condensation in the temperature and humidity sensor 16 even if the gas mixtures flow through the flow channel under a high-humidity environment. Consequently, the water vapor pressure of the gas mixtures may be measured more accurately even when the gas mixtures flow through the flow channel under the high-humidity environment.

(2) Configuration

A configuration for the physical quantity measurement system 1 will now be described in detail with reference to FIGS. 1-4.

The physical quantity measurement system 1 includes a flow channel body 10 and a processing device 20 as shown in FIG. 1. The physical quantity measurement system 1 measures, as physical quantities, the concentration of hydrogen (hereinafter simply referred to as a "hydrogen concentration") included in gas mixtures flowing through the flow channel body 10, the flow rate of the gas mixtures, and a relative humidity thereof. In addition, the physical quantity measurement system 1 also measures, as physical quantities, a water vapor pressure of water vapor included in the gas mixtures and the concentration of the water vapor (hereinafter simply referred to as a "water vapor concentration") with respect to the gas mixtures. In this case, the concentration measured by the physical quantity measurement system 1 may be, for example, a volume concentration.

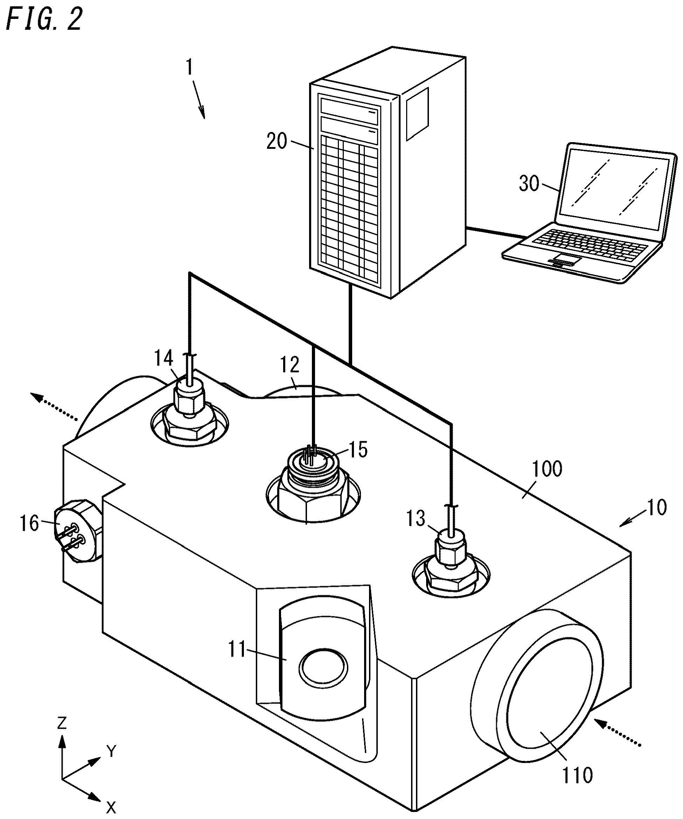

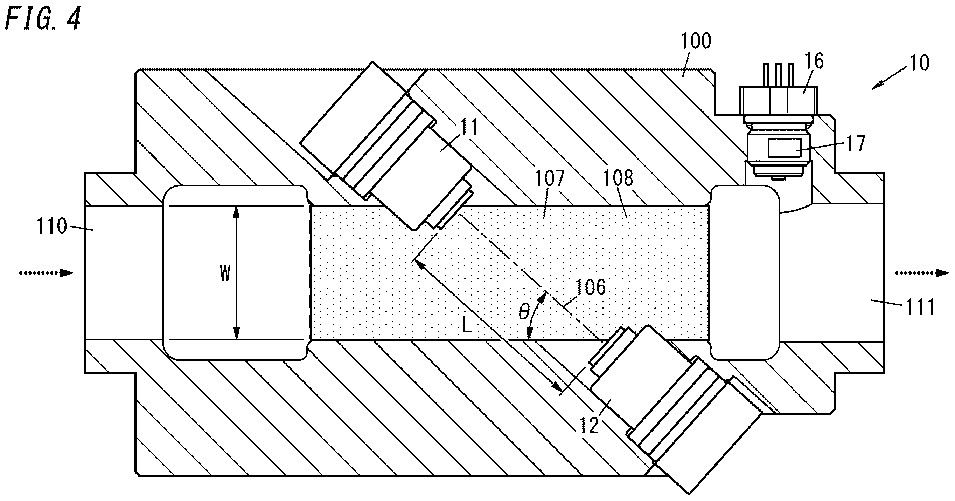

X-, Y-, and Z-axes are herein defined with respect to the flow channel body 10 (refer to FIG. 2). Specifically, the X-axis is herein defined to be an axis along the longitudinal axis of the flow channel body 10, i.e., an axis defined along a direction in which the gas mixtures flow. The Y-axis is an axis that intersects at right angles with the X-axis, and may be, for example, an axis defined along the depth of the flow channel body 10. The Z-axis is an axis that intersects at right angles with both the X- and Y-axes and may be, for example, an axis defined along the height of the flow channel body 10. FIG. 3 is a cross-sectional view, taken along an X-Z plane, of the flow channel body 10. FIG. 4 is a cross-sectional view, taken along an X-Y plane, of the flow channel body 10.

(2.1) Flow channel body

The gas mixtures flow through the flow channel body 10. The flow channel body 10 includes a pair of ultrasonic transducers 11, 12, a first temperature sensor 13, a second temperature sensor 14, a pressure sensor 15, a temperature and humidity sensor 16, a heat generator 17, and a switch unit 18 as shown in FIG. 1. The flow channel body 10 further includes a body 100 as shown in FIG. 2. Note that in the following description, the ultrasonic transducer 11 will be hereinafter sometimes referred to as a "first ultrasonic transducer 11" and the ultrasonic transducer 12 will be hereinafter sometimes referred to as a "second ultrasonic transducer 12."

The body 100 is formed in a substantially rectangular parallelepiped shape. A flow channel 101, through which a fluid under measurement (gas mixtures) such as a mixed gas including hydrogen flows, is provided to run through the middle of the body 100 (refer to FIG. 3). A first opening 110 and a second opening 111 are respectively provided at both longitudinal ends of the body 100. The first opening 110 and the second opening 111 are respectively provided through both side surfaces, facing each other along the longitudinal axis, of the body 100. The first opening 110 and the second opening 111 are connected to each other via the flow channel 101. The gas mixtures flow in through the first opening 110, flow through the flow channel 101, and flow out through the second opening 111.

The pair of ultrasonic transducers 11, 12 transmit and receive an ultrasonic wave. The pair of ultrasonic transducers 11, 12 are arranged to cause the ultrasonic wave to cross the flow of the gas mixtures along the flow channel 101. Specifically, the first ultrasonic transducer 11 transmits the ultrasonic wave toward the second ultrasonic transducer 12. In addition, the first ultrasonic transducer 11 also receives the ultrasonic wave transmitted from the second ultrasonic transducer 12. In the same way, the second ultrasonic transducer 12 transmits the ultrasonic wave toward the first ultrasonic transducer 11. In addition, the second ultrasonic transducer 12 also receives the ultrasonic wave transmitted from the first ultrasonic transducer 11. The first ultrasonic transducer 11 and the second ultrasonic transducer 12 are arranged at both lateral ends of the flow channel 101 and at upstream and downstream ends to cause an ultrasonic signal to cross the flow of the gas mixtures. The first ultrasonic transducer 11 and the second ultrasonic transducer 12 are also arranged on both side surfaces, facing each other along the lateral axis, of the flow channel 101 and at upstream and downstream ends to cause the ultrasonic signal to cross the flow of the gas mixtures. Specifically, the first ultrasonic transducer 11 and the second ultrasonic transducer 12 are arranged at upstream and downstream ends, respectively, to face each other (refer to FIGS. 2-4). In the direction in which the first ultrasonic transducer 11 and the second ultrasonic transducer 12 face each other, an ultrasonic wave propagation path 106 for propagating the ultrasonic wave therethrough is formed (refer to FIG. 4). The ultrasonic wave propagation path 106 forms a tilt angle $\theta$ with respect to the flow channel 101 (refer to FIG. 4).

The pressure sensor 15 is disposed in a central area of one principal surface (hereinafter referred to as an "upper surface") out of the two principal surfaces facing each other in a thickness direction defined with respect to the body 100 (refer to FIGS. 2 and 3). The pressure sensor 15 measures the pressure of the gas mixtures flowing through the flow channel 101.

The first temperature sensor 13 is arranged on the upper surface to be located opposite from the second opening 111 with respect to the pressure sensor 15 (refer to FIGS. 2 and 3). That is to say, the first temperature sensor 13 is disposed at an upstream end of the flow channel 101. The first temperature sensor 13 may be, for example, a thermocouple and measures the temperature of the gas mixtures flowing through the flow channel 101.

The second temperature sensor 14 is arranged on the upper surface to be located opposite from the first opening 110 with respect to the pressure sensor 15 (refer to FIGS. 2 and 3). That is to say, the second temperature sensor 14 is disposed at a downstream end of the flow channel 101. The second temperature sensor 14 may be, for example, a thermocouple and measures the temperature of the gas mixtures flowing through the flow channel 101.

The temperature and humidity sensor 16 includes a humidity sensor and a temperature sensor. That is to say, the temperature and humidity sensor 16 measures the humidity and temperature of the gas mixtures flowing through the flow channel 101. The temperature and humidity sensor 16 is provided for one side surface provided with the first ultrasonic transducer 11 out of two side surfaces facing each other along the lateral axis of the flow channel 101. The temperature and humidity sensor 16 is provided at a downstream end of the flow channel 101. Also, the first temperature sensor 13 and the second temperature sensor 14 and the temperature and humidity sensor 16 are arranged separately along the flow channel 101.

The heat generator 17 is provided for the temperature and humidity sensor 16. The temperature and humidity sensor 16 and the heat generator 17 are housed in the same housing 160. The heat generator 17 performs a heat generating operation of generating heat that warms the temperature and humidity sensor 16 (among other things, the humidity sensor thereof). The heat generator 17 is provided for the temperature and humidity sensor 16. Thus, the effect of the heat generated by the heat generator 17 on the second temperature sensor 14 is less significant than the effect of the heat generated by the heat generator 17 on the temperature and humidity sensor 16. In other words, the second temperature sensor 14 measures the temperature of the gas mixtures and is disposed at a place where the heat generated by the heat generator 17 affects the second temperature sensor 14 than the temperature and humidity sensor 16.

The switch unit 18 is provided on a power supply path between a power feeding unit 26 (refer to FIG. 1) and the temperature and humidity sensor 16 and the heat generator 17. The control unit 26 supplies electric power to the temperature and humidity sensor 16 and the heat generator 17. The switch unit 18 may be, for example, a metal-oxide semiconductor field effect transistor (MOSFET). Under the control of the processing device 20, the power supply path between: the power feeding unit 26; and the temperature and humidity sensor 16 and the heat generator 17 may be either cut off or the power feeding unit 26 may be electrically connected to the temperature and humidity sensor 16 and the heat generator 17 via the power supply path. No electric power is supplied to the temperature and humidity sensor 16 and the heat generator 17 any longer when the power supply path between: the power feeding unit 26; and the temperature and humidity sensor 16 and the heat generator 17 is cut off. This causes the temperature and humidity sensor 16 to stop measuring the temperature and humidity and reduces the heat generated by the heat generator 17. That is to say, the switch unit 18 is configured to be ready to cause the heat generator 17 to stop generating heat on detecting any abnormality in the heat generating operation by the heat generator 17.

The flow channel body 10 further includes one or more partitions 107. The one or more partitions 107 divides the flow channel 101 into multiple sections in the height direction H defined with respect to the flow channel 101. When the one or more partitions 107 divide the flow channel 101 into multiple sections, a multilayer flow channel 108 is formed in the flow channel 101. This configuration not only increases the aspect ratio of the flow channel cross section of each layer of the multilayer flow channel 108 to turn the flow into a two-dimensional flow but also reduces the Reynolds number to rectify the flow and stabilize the turbulence.

Note that the one or more partitions 107 are not indispensable constituent elements. That is to say, the flow channel body 10 (physical quantity measurement system 1) does not have to include the partition(s) 107.

(2.2) Processing device

The processing device 20 includes a first communications unit 21, a second communications unit 22, a third communications unit 23, a fourth communications unit 24, and a control unit 25 as shown in FIG. 1.

The processing device 20 includes a computer system including one or more processors and a memory, for example. The computer system performs the functions of the control unit 25 by making the processor execute a program stored in the memory. In this embodiment, the program to be executed by the processor is stored in advance in the memory. Alternatively, the program may also be distributed after having been stored in a non-transitory storage medium such as a memory card or downloaded via a telecommunications line such as the Internet.

The first communications unit 21 is a communications interface for communicating with the first ultrasonic transducer 11 and the second ultrasonic transducer 12. The second communications unit 22 is a communications interface for communicating with the first temperature sensor 13, the second temperature sensor 14, the pressure sensor 15, and the temperature and humidity sensor 16. The third communications unit 23 is a communications interface for communicating with the heat generator 17 and the switch unit 18. The fourth communications unit 24 is a communications interface for communicating with a user device 30 (refer to FIG. 2). The user device 30 is a device which includes a display unit such as a liquid crystal display to notify the user of, for example, the result of measurement made by the physical quantity measurement system 1.

The control unit 25 includes a first signal processor 201, a second signal processor 202, a heat generation controller 203, a water vapor pressure measurer 204, a concentration measurer 205, a flow rate measurer 206, and a relative humidity measurer 207 as shown in FIG. 1. The control unit 25 further includes a first detector 208 (environmental abnormality detector) and a second detector 209 (heat generation abnormality detector).

The first signal processor 201 performs processing concerning the communication between the first ultrasonic transducer 11 and the second ultrasonic transducer 12.

For example, when an ultrasonic wave is going to be transmitted from the first ultrasonic transducer 11 to the second ultrasonic transducer 12, the first signal processor 201 outputs a signal, instructing the first ultrasonic transducer 11 to transmit the ultrasonic wave, to the first ultrasonic transducer 11 via the first communications unit 21. When an ultrasonic wave is going to be transmitted from the second ultrasonic transducer 12 to the first ultrasonic transducer 11, the first signal processor 201 outputs a signal, instructing the second ultrasonic transducer 12 to transmit the ultrasonic wave, to the second ultrasonic transducer 12 the first communications unit 21.

In addition, the first signal processor 201 also measures a first propagation time (i.e., a propagation time of the ultrasonic wave in the forward direction) $t_{up}$ of the ultrasonic wave transmitted from the first ultrasonic transducer 11 to the second ultrasonic transducer 12. Specifically, the first signal processor 201 measures the first propagation time $t_{up}$ based on a time when the ultrasonic wave is transmitted from the first ultrasonic transducer 11 and a time when the ultrasonic wave transmitted from the first ultrasonic transducer 11 is received at the second ultrasonic transducer 12.

Furthermore, the first signal processor 201 also measures a second propagation time (i.e., a propagation time of the ultrasonic wave in the reverse direction) $t_{dw}$ of the ultrasonic wave transmitted from the second ultrasonic transducer 12 to the first ultrasonic transducer 11. Specifically, the first signal processor 201 measures the second propagation time $t_{dw}$ based on a time when the ultrasonic wave is transmitted from the second ultrasonic transducer 12 and a time when the ultrasonic wave transmitted from the second ultrasonic transducer 12 is received at the first ultrasonic transducer 11.

The second signal processor 202 receives the output signals of the respective sensors and subjects these signals received to predetermined processing. For example, on receiving the output signal of the first temperature sensor 13, the second signal processor 202 subjects the signal thus received to predetermined signal processing to obtain a first temperature value measured by the first temperature sensor 13. On receiving the output signal of the second temperature sensor 14, the second signal processor 202 subjects the signal thus received to predetermined signal processing to obtain a second temperature value measured by the second temperature sensor 14.

Furthermore, on receiving the output signal of the pressure sensor 15, the second signal processor 202 subjects the signal thus received to predetermined signal processing to obtain a pressure value measured by the pressure sensor 15.

On receiving the output signal of the humidity sensor of the temperature and humidity sensor 16, the second signal processor 202 subjects the signal thus received to predetermined signal processing to obtain a humidity value (humidity measured value) measured by the temperature and humidity sensor 16. On receiving the output signal of the temperature sensor of the temperature and humidity sensor 16, the second signal processor 202 subjects the signal thus received to predetermined signal processing to obtain a temperature value (temperature measured value) measured by the temperature and humidity sensor 16.

The heat generation controller 203 controls the ON/OFF states of the heat generator 17 to cause the heat generator 17 to change its state from an ON state where the heat generator 17 performs the heat generating operation to an OFF state where the heat generator 17 stops performing the heat generating operation, and vice versa. The heat generation controller 203 controls the ON/OFF states of the heat generator 17 based on the result of comparison between the humidity measured value of the gas mixtures that has been measured by the temperature and humidity sensor 16 and a first threshold value. That is to say, the heat generation controller 203 controls the heat generating operation by the heat generator 17 based on the result of comparison between the humidity measured value measured by the temperature and humidity sensor 16 and the first threshold value. As used herein, the "first threshold value" refers to a humidity value at which no condensation is produced in the temperature and humidity sensor 16. The first threshold value may be set at, for example, 70% as a humidity value at which no condensation is produced.

If the humidity measured value measured by the temperature and humidity sensor 16 is greater than the first threshold value (threshold value), then the heat generation controller 203 turns the heat generator 17 ON. On the other hand, if the humidity measured value measured by the temperature and humidity sensor 16 is equal to or less than the first threshold value, then the heat generation controller 203 turns the heat generator 17 OFF. Specifically, if the humidity measured value measured by the temperature and humidity sensor 16 is greater than the first threshold value), then the heat generation controller 203 controls, by pulse width modulation (PWM), a period in which the heat generator 17 turns ON and a period in which the heat generator 17 turns OFF, thereby alternately turning the heat generator 17 ON and OFF.

The heat generation controller 203 controls the pulse width of the pulse width modulation such that the greater the difference between the first threshold value and the humidity measured value is, the longer the ON state lasts. On the other hand, if the humidity measured value is equal to or less than the first threshold value, the heat generation controller 203 turns the heat generator 17 OFF. As used herein, the expression "the heat generator 17 is in ON state" refers to not only a situation where the heat generator 17 continues to be in ON state but also a situation where the heat generator 17 alternately turns ON and OFF under the control by pulse width modulation.

The water vapor pressure measurer 204 measures the water vapor pressure of water vapor included in the gas mixtures flowing through the flow channel. Irrespective of the state of the heat generator 17, the water vapor pressure measurer 204 measures the water vapor pressure $P_W$ of the water vapor based on the humidity measured value as a humidity value of the gas mixtures that has been measured by the temperature and humidity sensor 16 and the temperature measured value as a temperature value of the gas mixtures that has been measured by the temperature and humidity sensor 16. Specifically, the water vapor pressure measurer 204 measures the water vapor pressure $P_W$ by the following Mathematical Expression 1:

[Mathematical Expression 1]

$$P_W = 0.61 \cdot 10^{\frac{7.5 T_m}{T_m + 237.3}} \cdot \frac{H_m}{100}$$

where $T_m$ is a temperature measured value and $H_m$ is a humidity measured value.

In this case, the water vapor pressure measurer 204 calculates the water vapor pressure using a different measuring method depending on the condition satisfied based on the humidity measured value. Specifically, the water vapor pressure measurer 204 calculates the water vapor pressure $P_W$ by either the Mathematical Expression 1 or the following Mathematical Expression 2 depending on the condition satisfied based on the humidity measured value:

[Mathematical Expression 2]

$$P_W = 0.61 \cdot 10^{\frac{7.5 T_m}{T_m + 237.3}} \cdot \frac{H_m}{100} \cdot C_W$$

where $C_W$ is a correction value of the water vapor pressure and is a value equal to or less than 1.

In this embodiment, the water vapor pressure measurer 204 calculates the water vapor pressure $P_W$ by either a first measuring method or a second measuring method, which is different from the first measuring method, depending on the condition satisfied based on the humidity measured value. According to the first measuring method, the water vapor pressure $P_W$ is calculated based on the temperature measured value and the humidity measured value, i.e., by using Mathematical Expression 1. According to the second measuring method, the water vapor pressure $P_W$ is calculated based on the temperature measured value, the humidity measured value, and a correction value corresponding to the first threshold value, i.e., by using Mathematical Expression 2.

If the humidity measured value exceeds the first threshold value when Mathematical Expression 1 is used, then the water vapor pressure measurer 204 changes the equation for use to calculate the water vapor pressure $P_W$ from Mathematical Expression 1 into Mathematical Expression 2. That is to say, if the humidity measured value exceeds the first threshold value when Mathematical Expression 1 is used, then the water vapor pressure measurer 204 calculates the water vapor pressure $P_W$ using Mathematical Expression 2. On the other hand, if the humidity measured value is equal to or less than the first threshold value when Mathematical Expression 1 is used, the water vapor pressure measurer 204 does not change the equation for use to calculate the water vapor pressure $P_W$. That is to say, if the humidity measured value is equal to or less than the first threshold value when Mathematical Expression 1 is used, then the water vapor pressure measurer 204 calculates the water vapor pressure $P_W$ using Mathematical Expression 1. On the other hand, in this embodiment, if the humidity measured value is greater than the second threshold value when Mathematical Expression 2 is used, the water vapor pressure measurer 204 does not change the equation for use to calculate the water vapor pressure $P_W$. That is to say, if the humidity measured value is greater than the second threshold value when Mathematical Expression 2 is used, then the water vapor pressure measurer 204 calculates the water vapor pressure $P_W$ using Mathematical Expression 2. If the humidity measured value decreases to a value equal to or less than the second threshold value when Mathematical Expression 2 is used, then the water vapor pressure measurer 204 changes the equation for use to calculate the water vapor pressure $P_W$ from Mathematical Expression 2 into Mathematical Expression 1. That is to say, if the humidity measured value decreases to a value equal to or less than the second threshold value when Mathematical Expression 2 is used, then the water vapor pressure measurer 204 calculates the water vapor pressure $P_W$ using Mathematical Expression 1. In this case, the second threshold value is a value smaller than the first threshold value. For example, if the first threshold value is set at 70% as a humidity value at which no condensation is produced, then the second threshold value may be set at 60%.

That is to say, the water vapor pressure measurer 204 changes the threshold value for use to determine whether or not to change the measuring method, depending on whether the measuring state is a first state where the water vapor pressure $P_W$ is calculated using Mathematical Expression 1 or a second state where the water vapor pressure $P_W$ is calculated using Mathematical Expression 2. In the following description, the measuring method that uses Mathematical Expression 1 will be hereinafter referred to as a "first measuring method" and the measuring method that uses Mathematical Expression 2 will be hereinafter referred to as a "second measuring method."

If the humidity measured value exceeds the first threshold value when the measuring state is the first state where the first measuring method is used for measurement, then the water vapor pressure measurer 204 changes the measuring state from the first state into the second state where the second measuring method is used for measurement. On the other hand, if the humidity measured value is equal to or less than the first threshold value, then the water vapor pressure measurer 204 maintains the measuring state at the first state. In other words, if the humidity measured value exceeds the first threshold value when the measuring state is the first state, then the water vapor pressure measurer 204 changes the measuring method from the first measuring method into the second measuring method. On the other hand, if the humidity measured value is equal to or less than the first threshold value, then the water vapor pressure measurer 204 maintains the measuring method at the first measuring method.

If the humidity measured value decreases to a value equal to or less than the second threshold value that is smaller than the first threshold value when the measuring state is the second state, then the water vapor pressure measurer 204 changes the measuring state from the second state into the first state. On the other hand, if the humidity measured value is greater than the second threshold value, then the water vapor pressure measurer 204 maintains the measuring state at the second state. In other words, if the humidity measured value decreases to a value equal to or less than the second threshold value when the measuring state is the second state, then the water vapor pressure measurer 204 changes the measuring method from the second measuring method into the first measuring method. On the other hand, if the humidity measured value is greater than the second threshold value, then the water vapor pressure measurer 204 maintains the measuring method at the second measuring method.

The concentration measurer 205 measures, based on the water vapor pressure measured by the water vapor pressure measurer 204 and the pressure $P_m$ of the gas mixtures measured by the pressure sensor 15, a water vapor concentration $x_w$ as the concentration of water vapor included in the gas mixtures. Specifically, the concentration measurer 205 obtains the water vapor concentration $x_w$ using the following Mathematical Expression 3:

[Mathematical Expression 3]

$$x_w = P_W \cdot \frac{1}{P_m}$$

Furthermore, the concentration measurer 205 measures a gas concentration, which is the concentration of a gas included in the gas mixtures and different from the water vapor, based on the water vapor concentration thus obtained and the propagation time of the ultrasonic wave between the pair of ultrasonic transducers (namely, the first ultrasonic transducer 11 and the second ultrasonic transducer 12). For example, the concentration measurer 205 measures the concentration of hydrogen (hereinafter simply referred to as a "hydrogen concentration") included in the gas mixtures based on the water vapor concentration $x_w$ thus obtained and the propagation time of the ultrasonic wave. In this case, a speed of sound c of the gas mixtures, a molecular weight M of the gas mixtures, a heat capacity ratio $\gamma$ $(= c_p/c_v)$ of the gas mixtures, the temperature T of the gas mixtures, and a gas constant R satisfy the following mathematical expression 4:

[Mathematical Expression 4]

$$c^2 = \frac{\gamma RT}{M}$$

where $c_p$ indicates a molar heat capacity at constant pressure and $c_v$ indicates a molar specific heat at constant volume.

Suppose, in this case, the distance between the ultrasonic sensors (i.e., the length of the ultrasonic wave propagation path 106) is L. In that case, the speed of sound c is calculated based on the distance L and an average propagation time $t_{ave}$ of the first propagation time $t_{up}$ and the second propagation time $t_{dw}$ that have been measured. Thus, the following mathematical expression 5 is satisfied:

[Mathematical Expression 5]

$$c = \frac{L}{t_{ave}}$$

In this embodiment, hydrogen, nitrogen, and water vapor are included in the gas mixtures. Suppose, in this case, a molecular weight of hydrogen is $M_1$, a specific heat at constant pressure thereof is $c_{p1}$, and a specific heat at constant volume thereof is $c_{v1}$; a molecular weight of nitrogen is $M_2$, a specific heat at constant pressure thereof is $c_{p2}$, and a specific heat at constant volume thereof is $c_{v2}$; a molecular weight of water vapor is $M_w$, a specific heat at constant pressure thereof is $c_{pw}$, and a specific heat at constant volume thereof is $c_{vw}$; and the concentration of hydrogen is $x_1$. In that case, the following Mathematical Expressions 6 to 8 are satisfied:

[Mathematical Expression 6]

$$M = x_1 M_1 + (1 - x_1 - x_w)M_2 + x_w M_w$$

[Mathematical Expression 7]

$$c_p = x_1 c_{p1} + (1 - x_1 - x_w)c_{p2} + x_w c_{pw}$$

[Mathematical Expression 8]

$$c_v = x_1 c_{v1} + (1 - x_1 - x_w)c_{v2} + x_w c_{vw}$$

By using these Mathematical Expressions 6 to 8 and setting the temperature T at a temperature measured value $T_m$, Mathematical Expression 4 may be modified into the following Mathematical Expression 9:

[Mathematical Expression 9]

$$c^2 = \frac{\frac{x_1 c_{p1} + (1 - x_1 - x_w)c_{p2} + x_w c_{pw}}{x_1 c_{v1} + (1 - x_1 - x_w)c_{v2} + x_w c_{vw}}RT_m}{x_1 M_1 + (1 - x_1 - x_w)M_2 + x_w M_w}$$

The concentration measurer 205 obtains the hydrogen concentration $x_1$ using this Mathematical Expression 9.

Also, if the average of the actually measured propagation time of hydrogen is $t_{ave1}$, its temperature is $T_{m1}$, $x_1 = 1$, and $x_w = 0$ in Mathematical Expressions 5 and 9, then an actual distance L (= L1) between the ultrasonic sensors may be obtained. This allows the concentration of hydrogen to be measured highly accurately.

Furthermore, if the average propagation time is $t_{ave2}$ and the temperature is $T_{m2}$ when nitrogen is actually measured and $x_1 = 0$ and $x_w = 0$ in Mathematical Expressions 5 and 9, then the actual distance L (= L2) between the ultrasonic sensors may be obtained.

L1 = L2 is ideally satisfied. Actually, however, a difference may be caused between the distance L2 and the distance L1 due to the difference between a theoretical value and an actually measured value and owing to a fixed error involved with the conversion of the propagation time. In this case, the speed of sound is defined by the following Mathematical Expression 10 using a corrected propagation time ($t_{ave} - t_d$) calculated by subtracting the differential time $t_d$ from the propagation time $t_{ave}$.

[Mathematical Expression 10]

$$c = \frac{L}{t_{ave} - t_d}$$

By using this Mathematical Expression 10, $t_d$ and L may be determined to satisfy L1 = L2. In this case, $t_d$ and L may be obtained by numerical value analysis using $t_d$ = 0 as an initial value under the condition that L1 = L2 be satisfied. Alternatively, $t_d$ and L may also be obtained algebraically by the following Mathematical Expressions 11 and 12:

[Mathematical Expression 11]

$$\left(\frac{L_1}{t_{ave1} - t_d}\right)^2 = \frac{\frac{c_{p1}}{c_{v1}} RT_{m1}}{M_1}$$

[Mathematical Expression 12]

$$\left(\frac{L_2}{t_{ave2} - t_d}\right)^2 = \frac{\frac{c_{p2}}{c_{v2}} RT_{m2}}{M_2}$$

If $t_d$ and L thus obtained are used to make the calculations of Mathematical Expressions 9 and 10, then the concentration measurer 205 is allowed to measure the concentration more accurately.

The flow rate measurer 206 measures the flow rate of the gas mixtures in the flow channel 101 using the propagation time of the ultrasonic wave, the water vapor concentration, and the gas concentrations (including the hydrogen concentration and the nitrogen concentration). The flow rate measurer 206 obtains the flow rate Q of the gas mixtures using the following Mathematical Expressions 13 and 14. Mathematical Expression 13 is an equation for use to calculate a provisional flow rate $Q_0$. The provisional flow rate $Q_0$ is calculated by multiplying together a cross-sectional area S of the flow channel 101 and a flow velocity V. Also, based on the relationship between the flow velocity V, the length L of the ultrasonic wave propagation path 106 (i.e., the distance between the first ultrasonic transducer 11 and the second ultrasonic transducer 12), the first propagation time $t_{up}$, the second propagation time $t_{dw}$, and the angle θ formed by the ultrasonic wave propagation path 106 with respect to the flow channel 101, "$Q_0$ = SV" may be modified into the right side of Mathematical Expression 13, where $R_K$ is a flow coefficient:

[Mathematical Expression 13]

$$Q_0 = SV = \frac{SL}{2cos\theta}\left(\frac{1}{t_{up}} - \frac{1}{t_{dw}}\right)$$

[Mathematical Expression 14]

$$Q = R_K \cdot Q_0 = R_K \cdot \frac{SL}{2cos\theta}\left(\frac{1}{t_{up}} - \frac{1}{t_{dw}}\right)$$

Also, the flow coefficient $R_K$ is defined by the following Mathematical Expression 15 using a kinematic viscosity v, a characteristic length D, and a flow velocity $V_m$. In Mathematical Expression 15, $R_e$ indicates the Reynolds number. That is to say, the Reynolds number $R_e$ is given by the equation "$R_e = V_m D/v$." Note that the characteristic length D may be, for example, either the length between a pair of partitions 107 or the length between one sidewall, which is one of two sidewalls facing each other in the height direction H which form the flow channel 101, and a partition 107 located closest to the one sidewall. If no partitions 107 are provided, then the characteristic length D may be equal to the height H, for example. Also, the function f ($R_e$) is a predefined function. The flow velocity $V_m$ is a flow velocity corresponding to a flow rate (hereinafter referred to as a "reference flow rate") which has been measured by a calibrator during a calibration process performed as advance evaluation.

[Mathematical Expression 15]

$$R_K = f(R_e) = f\left(\frac{V_m D}{v}\right)$$

In this case, the kinematic viscosity v is a value which depends on the temperature T in the flow channel 101, the concentrations of the respective gases (namely, hydrogen, nitrogen, and water vapor) included in the gas mixtures, and the pressure $P_m$. In addition, since the kinematic viscosity v satisfies the equation "kinematic viscosity = viscosity/density," the kinematic viscosity v may be given by the following Mathematical Expression 16. In Mathematical Expression 16, the middle side is modified into the right side in accordance with Relational Expression #2 "density = (molecular weight M · pressure $P_m$) / (gas constant R·temperature T)" derived from the state equation of a gas "pressure $P_m$·volume = number of moles ·gas constant R·temperature T" and Relational Expression #1 "density = mass/volume." That is to say, the density ρ may be expressed by the molecular weight M, the pressure $P_m$, the temperature T, and the gas constant R as can be seen from the state equation of the gas and Relational Expression #1. In this case, the pressure $P_m$ is a pressure measured by the pressure sensor 15. The temperature T is an average value of a first temperature value measured by the first temperature sensor 13 and a second temperature value measured by the second temperature sensor 14. Also, the density ρ is determined by the concentration, temperature, and pressure of the gas. Thus, in Relational Expression #2, the molecular weight M is a function which uses concentration as a variable (refer to Mathematical Expression 6).

[Mathematical Expression 16]

$$v(x, T, P) = \frac{\mu(x, T)}{\rho(x, T, P_m)} = \frac{\mu(x, T)RT}{M(x)P_m}$$

In this Mathematical Expression 16, μ (x, T) may be determined based on the viscosities of the respective gases (namely, hydrogen, nitrogen, and water vapor) included in the gas mixtures. The viscosities of the respective gases may be obtained by a known method. The viscosity μ of the gas mixtures may be determined by using the viscosities of the respective gases obtained by the known method and the concentrations of the respective gases (i.e., the hydrogen concentration, the nitrogen concentration, and the water vapor concentration) that have already been obtained.

The flow rate measurer 206 obtains, based on the flow rate Q of the gas mixtures thus obtained, a standard flow rate $Q_n$ of the gas mixtures which is converted into a value corresponding to the condition including 0°C and 1 atm.

The flow rate measurer 206 measures the flow rate of a gas using the flow rate of the gas mixtures. Specifically, the flow rate measurer 206 measures the flow rate of the gas by multiplying the standard flow rate $Q_n$ that has been obtained based on the flow rate Q of the gas mixtures by the concentration of the gas. The flow rate measurer 206 obtains the flow rate of hydrogen by multiplying the standard flow rate $Q_n$ by the hydrogen concentration $x_1$ as a gas concentration. Alternatively, the flow rate measurer 206 may obtain the flow rate of hydrogen by multiplying the flow rate Q by the hydrogen concentration $x_1$ as a gas concentration.

Next, it will be described how to obtain the flow coefficient $R_K$.

The flow coefficient $R_K$ is expressed by the following Mathematical Expression 17 using a provisional flow rate $Q_0$ and a reference flow rate $Q_m$, where the reference flow rate $Q_m$ is a flow rate measured by a calibrator at the time of calibration.

[Mathematical Expression 17]

$$R_K = \frac{Q_m}{Q_0}$$

Also, the Reynolds number $R_e$ is expressed by the following Mathematical Expression 18 using the reference flow rate $Q_m$. In Mathematical Expression 18, v indicates the kinematic viscosity of the gas mixtures, D indicates the characteristic length, and S indicates a cross-sectional area of the flow channel 101. $V_m$ is a flow velocity corresponding to the reference flow rate $Q_m$ measured by a calibrator at the time of calibration.

[Mathematical Expression 18]

$$R_e = \frac{V_m D}{\nu} = \frac{V_m S D}{\nu S} = \frac{Q_m D}{\nu S}$$

The processing device 20 calculates, using Mathematical Expressions 17 and 18, multiple combinations ($R_e$, $R_K$), each of which consists of the Reynolds number $R_e$ in a situation where the reference flow rate $Q_m$ has been changed and the flow coefficient $R_K$ that has been obtained by using the provisional flow rate $Q_0$ derived from a measured value, as advance evaluation. The processing device 20 obtains a relational expression between the Reynolds number $R_e$ and the flow coefficient $R_K$ based on the result of calculation and stores the relational expression in advance.

In addition, the following Mathematical Expression (19) is also obtained as a relational expression between the Reynolds number $R_e$ and the flow coefficient $R_K$ by using Mathematical Expressions 17 and 18. The characteristic length D and the cross-sectional area S are known values for the physical quantity measurement system 1, and the kinematic viscosity $\nu$ and the provisional flow rate $Q_0$ are values which have already been calculated. That is to say, the flow coefficient $R_K$ is expressed as a linear function which uses the Reynolds number $R_e$. In other words, Mathematical Expression (19) is a linear function representing the relationship between the Reynolds number $R_e$ and the flow coefficient $R_K$ and is a function which uses, as a coefficient, a value $((\nu S)/(Q_0 D))$ based on the provisional flow rate $Q_0$ and the kinematic viscosity $\nu$ of the gas mixtures.

[Mathematical Expression 19]

$$R_K = \frac{\nu S}{Q_0 D} R_e$$

The flow rate measurer 206 calculates the flow coefficient $R_K$ using a relational expression representing the relationship between the Reynolds number $R_e$ and the flow coefficient $R_K$ which have been obtained by advance evaluation and Mathematical Expression (19). Specifically, the flow rate measurer 206 calculates a flow coefficient $R_K$ corresponding to the provisional flow rate $Q_0$ by locating an intersection between a graph represented by the relational expression obtained by advance evaluation and a graph expressed by Mathematical Expression (19).

The flow rate measurer 206 calculates the flow rate Q using the flow coefficient $R_K$ thus calculated and Mathematical Expression 14.

In this case, the kinematic viscosity $\nu$ has appeared in both Mathematical Expressions 15 and 18. That is to say, the flow coefficient $R_K$ according to the present disclosure is a value based on the kinematic viscosity $\nu$ of the gas mixtures. Also, according to Mathematical Expression 18, the Reynolds number $R_e$ uses the kinematic viscosity $\nu$ as a coefficient. That is to say, the flow coefficient $R_K$ is a value obtained by using a linear function of the Reynolds number $R_e$ that uses, as a coefficient, a value based on the kinematic viscosity $\nu$ of the gas mixtures. More specifically, the flow coefficient $R_K$ is a value obtained based on the linear function of the Reynolds number that uses, as a coefficient, a value based on the kinematic viscosity $\nu$ of the gas mixtures and the relational expression representing the relationship between the Reynolds number and the flow coefficient which has been obtained by advance evaluation.

Alternatively, the processing device 20 may also use, instead of the relational expression obtained by advance evaluation, a data table including multiple combinations ($R_e$, $R_k$), each of which consists of the Reynolds number $R_e$ and the flow coefficient $R_K$ that have been calculated by advance evaluation.

The relative humidity measurer 207 measures a relative humidity of the gas mixtures based on the water vapor pressure $P_W$ measured by the water vapor pressure measurer 204 and a saturated water vapor pressure $P_{WS}$ corresponding to the temperature (i.e., the temperature of the gas mixtures) measured by the second temperature sensor 14. More specifically, the relative humidity measurer 207 measures the relative humidity $H_0$ of the gas mixtures based on the humidity measured value $H_m$ and temperature measured value $T_m$ measured by the temperature and humidity sensor 16 and the temperature (second temperature value) of the gas mixtures that has been measured by the second temperature sensor 14. Specifically, the relative humidity measurer 207 obtains the relative humidity of the gas mixtures by one of the following Mathematical Expressions 20 and 21. If the measuring state is the first state, the relative humidity measurer 207 obtains the relative humidity of the gas mixtures using Mathematical Expression 20. If the measuring state is the second state, the relative humidity measurer 207 obtains the relative humidity of the gas mixtures using Mathematical Expression 21. In Mathematical Expressions 20 and 21, $P_{WS}$ is a saturated water vapor pressure with respect to the temperature measured by the second temperature sensor 14. That is to say, $P_{WS}$ is a saturated water vapor pressure with respect to the temperature of the gas mixtures. Also, $T_n$ is a temperature (second temperature value) measured by the second temperature sensor 14.

[Mathematical Expression 20]

$$H_0 = (P_W/P_{WS}) \cdot 100 = (P_W/(0.61 \cdot 10^{\frac{7.5T_n}{T_n+237.3}})) \cdot 100 = H_m \cdot 10^{\left(\frac{7.5T_m}{T_m+237.3} - \frac{7.5T_n}{T_n+237.3}\right)}$$

[Mathematical Expression 21]

$$H_0 = (P_W/P_{WS}) \cdot 100 = (P_W/(0.61 \cdot 10^{\frac{7.5T_n}{T_n+237.3}})) \cdot 100 = H_m \cdot C_W \cdot 10^{\left(\frac{7.5T_m}{T_m+237.3} - \frac{7.5T_n}{T_n+237.3}\right)}$$

Alternatively, if the measuring state is the first state, the relative humidity measurer 207 may regard the humidity measured value $H_m$ as the relative humidity $H_0$ of the gas mixtures.

The first detector 208 detects any abnormality in a measuring environment for the temperature and humidity sensor 16 in a situation where the heat generator 17 is in ON state. That is to say, the first detector 208 serves as an environmental abnormality detector for detecting any abnormality in the measuring environment for the temperature and humidity sensor 16 in the situation where the heat generator 17 is in ON state. The first detector 208 obtains a standard deviation σ of the humidity measured value based on the humidity measured value measured by the temperature and humidity sensor 16 in the situation where the heat generator 17 is in ON state. In this case, the standard deviation σ obtained by the first detector 208 is a standard deviation in a predetermined period in the situation where the heat generator 17 is in ON state. The first detector 208 determines a reference value corresponding to the water vapor pressure $P_W$ measured by the water vapor pressure measurer 204. For example, the first detector 208 acquires (determines), as the reference value, a predetermined value corresponding to the water vapor pressure $P_W$ measured by the water vapor pressure measurer 204 which belongs to a plurality of predetermined values respectively corresponding to a plurality of water vapor pressures that are stored in advance in a memory. If the standard deviation σ is greater than the reference value, then the first detector 208 detects the occurrence of abnormality in the situation where the heat generator 17 is in ON state. In this embodiment, the first detector 208 detects, as abnormality of the measuring environment for the temperature and humidity sensor 16, that the temperature and humidity sensor 16 has sunk in water.

On detecting that any abnormality has occurred in the situation where the heat generator 17 is in ON state, the first detector 208 outputs a first detection result indicating that the abnormality has occurred to the measuring environment for the temperature and humidity sensor 16 to the user device 30 via the fourth communications unit 24.

The second detector 209 detects any abnormality in the heat generating operation by the heat generator 17. That is to say, the second detector 209 serves as a heat generation abnormality detector for detecting any abnormality in the heat generating operation. If the heat generating operation is performed by the heat generator 17 and the humidity measured value of the gas mixtures and the temperature measured value of the gas mixtures that have been measured by the temperature and humidity sensor 16 satisfy a predetermined condition, then the second detector 209 detects the occurrence of abnormality. That is to say, the second detector 209 detects the occurrence of abnormality if the humidity measured value of the gas mixtures and the temperature measured value of the gas mixtures that have been measured by the temperature and humidity sensor 16 satisfy the predetermined condition while the heat generator 17 is performing the heat generating operation. Specifically, if the result (first differential value) calculated by subtracting the humidity measured value from the first threshold value is greater than a first comparison value, the result (second differential value) calculated by subtracting the temperature (second temperature value) measured by the second temperature sensor 14 from the temperature measured value is greater than a second comparison value, and the temperature measured value is greater than a predetermined third comparison value, then the second detector 209 determines that the predetermined condition be satisfied and detects the occurrence of abnormality in the heat generating operation.

In this case, the abnormality detected by the second detector 209 may be a communication error caused while a signal instructing the heat generator 17 to stop performing the heat generating operation is being transmitted. Alternatively, the abnormality detected by the second detector 209 may also be an internal malfunction of the physical quantity measurement system 1. The internal malfunction of the physical quantity measurement system 1 may be, for example, a failure of the heat generator 17.

On detecting that any abnormality has occurred, the second detector 209 outputs a second detection result indicating that the abnormality has occurred to the heat generating operation to the user device 30 via the fourth communications unit 24.

On detecting the occurrence of the abnormality to the heat generating operation, the second detector 209 outputs a cutoff instruction signal, instructing that the power supply path between the power feeding unit 26 and the temperature and humidity sensor 16 and the heat generator 17 be cut off, to the switch unit 18 via the third communications unit 23. On receiving the cutoff instruction signal, the switch unit 18 cuts off the power supply path between the power feeding unit 26 and the temperature and humidity sensor 16 and the heat generator 17. In this manner, the heat generating operation by the heat generator 17 may be suspended.

If the result (first differential value) calculated by subtracting the humidity measured value from the first threshold value is greater than the first comparison value and the result (second differential value) calculated by subtracting the temperature (second temperature value) measured by the second temperature sensor 14 from the temperature measured value is greater than the second comparison value, then the second detector 209 may determine that the predetermined condition be satisfied and detect the occurrence of abnormality to the heat generating operation.

(3) Operation

In this section, it will be described how the physical quantity measurement system 1 operates.

(3.1) Overview of operation

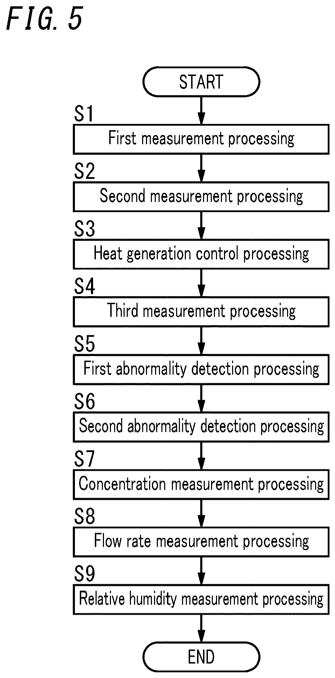

First, an overview of the operation of the physical quantity measurement system 1 will be described with reference to FIG. 5.

The first signal processor 201 performs first measurement processing (in Step S1). Specifically, the first signal processor 201 measures a first propagation time $t_{up}$ of the ultrasonic wave transmitted from the first ultrasonic transducer 11 to the second ultrasonic transducer 12. In addition, the first signal processor 201 also measures a second propagation time $t_{dw}$ of the ultrasonic wave transmitted from the second ultrasonic transducer 12 to the first ultrasonic transducer 11.

The second signal processor 202 performs second measurement processing (in Step S2). Specifically, the second signal processor 202 obtains, based on the output signal of the first temperature sensor 13, a first temperature value measured by the first temperature sensor 13. In addition, the second signal processor 202 also obtains, based on the output signal of the second temperature sensor 14, a second temperature value measured by the second temperature sensor 14. Furthermore, the second signal processor 202 further obtains, based on the output signal of the pressure sensor 15, a pressure value measured by the pressure sensor 15. Furthermore, the second signal processor 202 further obtains, based on the output signal of the humidity sensor of the temperature and humidity sensor 16, a humidity value (humidity measured value) measured by the temperature and humidity sensor 16 and a temperature value (temperature measured value) measured by the temperature and humidity sensor 16.

The heat generation controller 203 performs heat generation control processing (in Step S3). The heat generation controller 203 controls the ON/OFF states of the heat generator 17 to cause the heat generator 17 to change its state from an ON state where the heat generator 17 performs the heat generating operation to an OFF state where the heat generator 17 stops performing the heat generating operation, and vice versa.

The water vapor pressure measurer 204 performs third measurement processing (in Step S4). The water vapor pressure measurer 204 performs the third measurement processing to calculate a water vapor pressure and a water vapor concentration.

The first detector 208 performs first abnormality detection processing (in Step S5). The first detector 208 performs the first abnormality detection processing to detect any abnormality in the measuring environment for the temperature and humidity sensor 16.

The second detector 209 performs second abnormality detection processing (in Step S6). The second detector 209 performs the second abnormality detection processing to detect any abnormality in the heat generating operation by the heat generator 17.

The concentration measurer 205 performs concentration measurement processing (in Step S7). The concentration measurer 205 performs concentration measurement processing to calculate the concentration of hydrogen flowing through the flow channel 101.

The flow rate measurer 206 performs flow rate measurement processing (in Step S8). The flow rate measurer 206 performs the flow rate measurement processing to calculate the flow rate of the gas mixtures flowing through the flow channel 101 and the flow rate of hydrogen included in the gas mixtures.

The relative humidity measurer 207 performs relative humidity measurement processing (in Step S9). The relative humidity measurer 207 performs the relative humidity measurement processing to calculate the relative humidity of the gas mixtures flowing through the flow channel 101.

The (control unit 25 of the) processing device 20 notifies, via the fourth communications unit 24, the user device 30 of at least one of the flow rate, concentration, or relative humidity of the gas mixtures thus calculated.

(3.2) Heat generation control processing

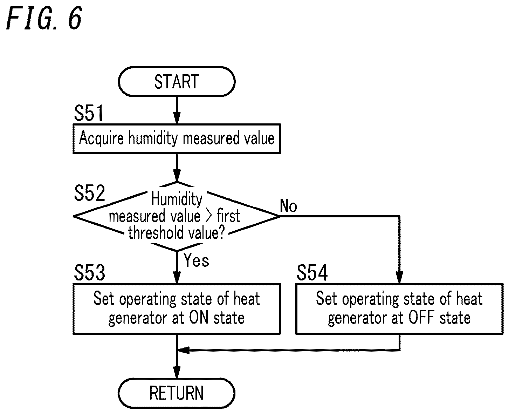

In this section, the heat generation control processing in Step S3 shown in FIG. 5 will be described with reference to FIG. 6.

The heat generation controller 203 acquires the humidity measured value obtained by the second signal processor 202 (in Step S51).

The heat generation controller 203 determines whether or not the humidity measured value thus acquired is greater than the first threshold value (in Step S52).

If the heat generation controller 203 determines that the humidity measured value be greater than the first threshold value (if the answer is Yes in Step S52), the heat generation controller 203 sets the operating state of the heat generator 17 at ON state (in Step S53). Specifically, if the humidity measured value is greater than the first threshold value, the heat generation controller 203 controls, by pulse width modulation, a period in which the heat generator 17 turns ON and a period in which the heat generator 17 turns OFF, thereby alternately turning the heat generator 17 ON and OFF. The heat generation controller 203 controls the pulse width of the pulse width modulation such that the greater the difference between the first threshold value and the humidity measured value is, the longer the ON state becomes.

On the other hand, if the heat generation controller 203 determines that the humidity measured value be not greater than the first threshold value, i.e., the humidity measured value be equal to or less than the first threshold value (if the answer is No in Step S52), the heat generation controller 203 turns the heat generator 17 OFF.

(3.3) Third measurement processing

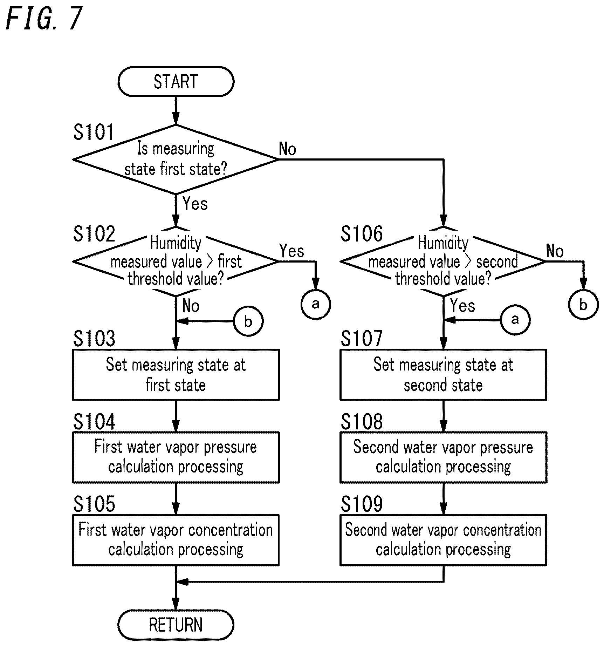

In this section, the third measurement processing in Step S4 shown in FIG. 5 will be described with reference to FIG. 7.

The water vapor pressure measurer 204 determines whether or not the measuring state is the first state (in Step S101).

If the water vapor pressure measurer 204 determines the measuring state to be the first state (if the answer is Yes in Step S101), then the water vapor pressure measurer 204 determines whether or not the humidity measured value obtained by the second signal processor 202 is greater than the first threshold value (in Step S102).

When determining that the humidity measured value not be greater than the first threshold value, i.e., when determining that the humidity measured value be equal to or less than the first threshold value (if the answer is No in Step S102), the water vapor pressure measurer 204 sets the measuring state at the first state (in Step S103). After having set the measuring state at the first state in Step S103, the water vapor pressure measurer 204 performs first water vapor pressure calculation processing (in Step S104). Specifically, the water vapor pressure measurer 204 calculates the water vapor pressure $P_W$ by the Mathematical Expression 1 described above. Thereafter, the concentration measurer 205 performs first water vapor concentration calculation processing (in Step S105). The concentration measurer 205 measures the water vapor concentration $x_w$ of the water vapor included in the gas mixtures using the water vapor pressure $P_W$ obtained in Step S104, the pressure $P_m$ of the gas mixtures that has been obtained by the second signal processor 202, and the Mathematical Expression 3 described above.

When determining that the measuring state not be the first state, i.e., when determining the measuring state to be the second state (if the answer is No in Step S101), the water vapor pressure measurer 204 determines whether or not the humidity measured value obtained by the second signal processor 202 is greater than the second threshold value (in Step S106).

When determining that the humidity measured value be greater than the second threshold value (if the answer is Yes in Step S106), the water vapor pressure measurer 204 sets the measuring state at the second state (in Step S107). After having set the measuring state at the second state in Step S107, the water vapor pressure measurer 204 performs second water vapor pressure calculation processing (in Step S108). Specifically, the water vapor pressure measurer 204 calculates the water vapor pressure $P_W$ by the Mathematical Expression 2 described above. Thereafter, the concentration measurer 205 performs second water vapor concentration calculation processing (in Step S109). The concentration measurer 205 measures the water vapor concentration $x_w$ of the water vapor included in the gas mixtures using the water vapor pressure $P_W$ obtained in Step S108, the pressure $P_m$ of the gas mixtures that has been obtained by the second signal processor 202, and the Mathematical Expression 3 described above.

When determining in Step S102 that the humidity measured value be greater than the first threshold value (if the answer is Yes in Step S102), the water vapor pressure measurer 204 sets the measuring state at the second state (in Step S107). After having set the measuring state at the second state in Step S107, the water vapor pressure measurer 204 performs second water vapor pressure calculation processing (in Step S108). After that, the concentration measurer 205 performs second water vapor concentration calculation processing (in Step S109).

When determining in Step S106 that the humidity measured value not be greater than the second threshold value, i.e., when determining that the humidity measured value be equal to or less than the second threshold value (if the answer is No in Step S106), the water vapor pressure measurer 204 sets the measuring state at the first state (in Step S103). After having set the measuring state at the first state in Step S103, the water vapor pressure measurer 204 performs first water vapor pressure calculation processing (in Step S104). After that, the concentration measurer 205 performs first water vapor concentration calculation processing (in Step S105).

(3.4) First abnormality detection processing

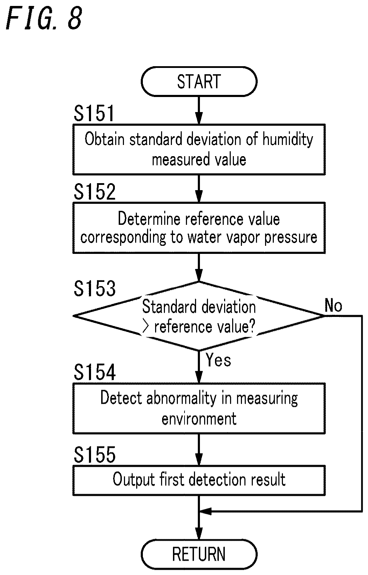

In this section, the first abnormality detection processing in Step S5 shown in FIG. 5 will be described with reference to FIG. 8.

The first detector 208 obtains a standard deviation $\sigma$ of the humidity measured value based on the humidity measured value measured by the temperature and humidity sensor 16 in the situation where the heat generator 17 is in ON state (in Step S151).

The first detector 208 determines a reference value corresponding to the water vapor pressure $P_W$ measured by the water vapor pressure measurer 204 (in Step S152). For example, the first detector 208 acquires (determines), as the reference value, a predetermined value corresponding to the water vapor pressure $P_W$ measured by the water vapor pressure measurer 204 which belongs to a plurality of predetermined values respectively corresponding to a plurality of water vapor pressures.

The first detector 208 determines whether or not the standard deviation $\sigma$ is greater than the reference value (in Step S153).

If the first detector 208 determines that the standard deviation $\sigma$ not be greater than the reference value (if the answer is No in Step S153), then the processing ends. On the other hand, if the first detector 208 determines that the standard deviation o be greater than the reference value (if the answer is Yes in Step S153), then the first detector 208 detects the occurrence of abnormality of the measuring environment for the temperature and humidity sensor 16 in the situation where the heat generator 17 is in ON state (in Step S154). For example, the first detector 208 may detect, as abnormality of the measuring environment for the temperature and humidity sensor 16, that the temperature and humidity sensor 16 has sunk in water.

On detecting that any abnormality has occurred to the measuring environment for the temperature and humidity sensor 16 in the situation where the heat generator 17 is in ON state, the first detector 208 outputs a first detection result indicating that the abnormality has occurred to the measuring environment for the temperature and humidity sensor 16 to the user device 30 (in Step S155).

(3.5) Second abnormality detection processing

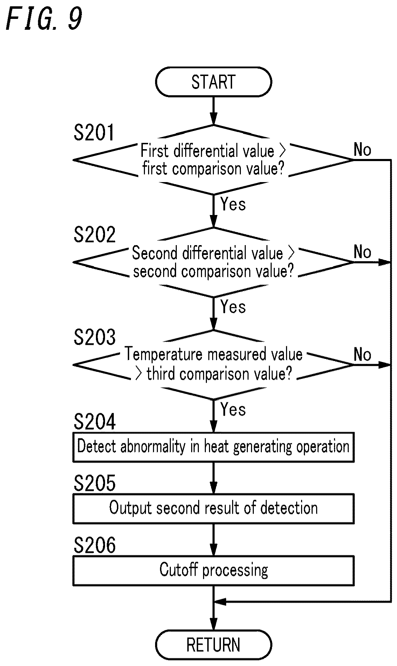

In this section, the second abnormality detection processing in Step S6 shown in FIG. 5 will be described with reference to FIG. 9.

The second detector 209 determines whether or not the result (first differential value) calculated by subtracting the humidity measured value from the first threshold value is greater than a first comparison value (in Step S201).

If the second detector 209 determines that the first differential value not be greater than the first comparison value (if the answer is No in Step S201), the processing ends. On the other hand, if the second detector 209 determines that the first differential value be greater than the first comparison value (if the answer is Yes in Step S201), then the second detector 209 determines whether or not the result (second differential value) calculated by subtracting the temperature (second temperature value) measured by the second temperature sensor 14 from the temperature measured value is greater than a second comparison value (in Step S202).

If the second detector 209 determines that the second differential value not be greater than the second comparison value (if the answer is No in Step S202), the processing ends. On the other hand, if the second detector 209 determines that the second differential value be greater than the second comparison value (if the answer is Yes in Step S202), then the second detector 209 determines whether or not the temperature measured value is greater than a third comparison value (in Step S203).

If the second detector 209 determines that the temperature measured value not be greater than the third comparison value (if the answer is No in Step S203), the processing ends. On the other hand, if the second detector 209 determines that the temperature measured value be greater than the third comparison value (if the answer is Yes in Step S203), then the second detector 209 determines that the predetermined condition be satisfied and detects the occurrence of abnormality to the heat generating operation (in Step S204).

On detecting that any abnormality has occurred to the heat generating operation, the second detector 209 outputs a second detection result indicating that the abnormality has occurred to the heat generating operation to the user device 30 (in Step S205).

On detecting the occurrence of the abnormality to the heat generating operation, the second detector 209 performs cutoff processing (in Step S206). The second detector 209 outputs a cutoff instruction signal to the switch unit 18 via the third communications unit 23. On receiving the cutoff instruction signal, the switch unit 18 cuts off the power supply path between: the power feeding unit 26; and the temperature and humidity sensor 16 and the heat generator 17. In this manner, the heat generating operation by the heat generator 17 may be suspended.

(3.6) Concentration measurement processing

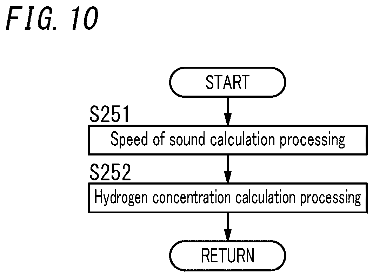

In this section, the concentration measurement processing in Step S7 shown in FIG. 5 will be described with reference to FIG. 10.

The concentration measurer 205 performs the concentration measurement processing to measure a gas concentration, i.e., the concentration of a gas (e.g., hydrogen in this example) included in the gas mixtures and different from water vapor.

The concentration measurer 205 performs speed of sound calculation processing (in Step S251). The concentration measurer 205 calculates the speed of sound based on the propagation time of the ultrasonic wave.

The concentration measurer 205 performs hydrogen concentration calculation processing (in Step S252). The concentration measurer 205 calculates the concentration of hydrogen using the water vapor concentration $x_w$ that has been obtained in either the first water vapor concentration calculation processing or second water vapor concentration calculation processing of the third measurement processing and the speed of sound.

(3.7) Speed of sound calculation processing

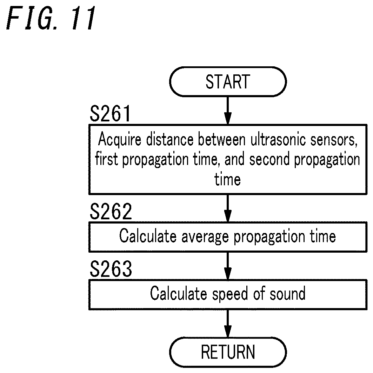

In this section, the speed of sound measurement processing in Step S251 shown in FIG. 10 will be described with reference to FIG. 11.

The concentration measurer 205 acquires the distance L (i.e., the distance between the first ultrasonic transducer 11 and the second ultrasonic transducer 12) that has been stored in advance and the first propagation time $t_{up}$ and second propagation time $t_{dw}$ that have been measured by the first signal processor 201 (in Step S261).

The concentration measurer 205 calculates an average propagation time $t_{ave}$ using the first propagation time $t_{up}$ and the second propagation time $t_{dw}$ (in Step S262).

The concentration measurer 205 calculates the speed of sound using the distance L acquired in Step S261, the average propagation time $t_{ave}$ calculated in Step S262, and the Mathematical Expression 5 described above (in Step S263).

(3.8) Hydrogen concentration calculation processing

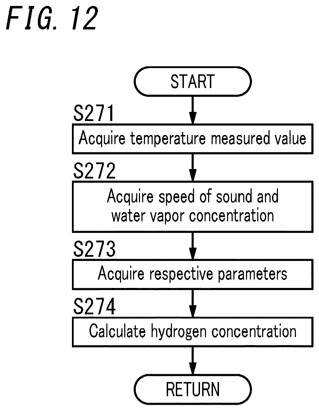

In this section, the hydrogen concentration calculation processing in Step S252 shown in FIG. 10 will be described with reference to FIG. 12.

The concentration measurer 205 acquires the temperature measured value $T_m$ obtained by the second signal processor 202 (in Step S271).

The concentration measurer 205 acquires the speed of sound c calculated in the speed of sound processing and the water vapor concentration $x_w$ calculated in the third measurement processing (in Step S272).

The concentration measurer 205 acquires the respective parameters that have been stored in advance (in Step S273). Specifically, the concentration measurer 205 acquires, as respective parameters, a gas constant R, the molecular weight $M_1$ of hydrogen, the molecular weight $M_2$ of nitrogen, the molecular weight $M_w$ of water vapor, respective specific heat values at constant volume of the respective gases (namely, hydrogen, nitrogen, and water vapor), and specific heat values at constant volume of the respective gases (namely, hydrogen, nitrogen, and water vapor).

The concentration measurer 205 calculates the hydrogen concentration $x_1$ (in Step S274). Specifically, the concentration measurer 205 calculates the hydrogen concentration $x_1$ using the speed of sound c, the temperature measured value $T_m$, the gas constant R, the water vapor concentration $x_w$, the molecular weight $M_1$ of hydrogen, the molecular weight $M_2$ of nitrogen, the molecular weight $M_w$ of water vapor, respective specific heat values at constant volume of the respective gases (namely, hydrogen, nitrogen, and water vapor), specific heat values at constant volume of the respective gases (namely, hydrogen, nitrogen, and water vapor), and Mathematical Expression 9. That is to say, the concentration measurer 205 calculates the hydrogen concentration x1 using the speed of sound c, the temperature measured value $T_m$, the gas constant R, the molecular weight M of the gas mixtures, the heat capacity ratio $\gamma$ (= $c_p/c_v$) of the gas mixtures, and Mathematical Expression 4.

(3.9) Flow rate measurement processing

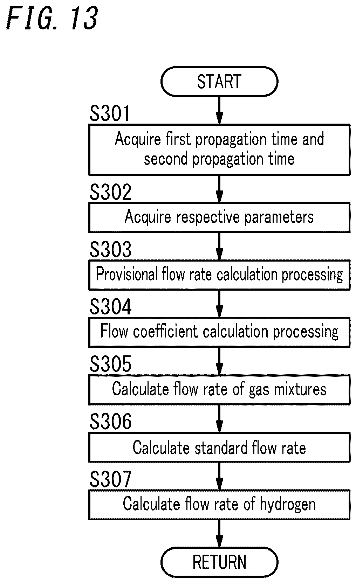

In this section, the flow rate measurement processing in Step S8 shown in FIG. 5 will be described with reference to FIG. 13.

The flow rate measurer 206 acquires the first propagation time $t_{up}$ and second propagation time $t_{dw}$ that have been measured by the first signal processor 201 (in Step S301).

The flow rate measurer 206 acquires the respective parameters that have been stored in advance (in Step S302). Specifically, the flow rate measurer 206 acquires, as the respective parameters, the cross-sectional area S of the flow channel 101, the length L of the ultrasonic wave propagation path 106 (i.e., the distance L between the first ultrasonic transducer 11 and the second ultrasonic transducer 12), and the tilt angle $\theta$ defined by the ultrasonic wave propagation path 106 with respect to the flow channel 101.

The flow rate measurer 206 performs provisional flow rate calculation processing (in Step S303). Specifically, the flow rate measurer 206 calculates the provisional flow rate $Q_0$ using the first propagation time $t_{up}$ and second propagation time $t_{dw}$ that have been acquired in Step S301, the cross-sectional area S, length L, and angle $\theta$ that have been acquired in Step S302, and Mathematical Expression 13 (in Step S303).

The flow rate measurer 206 performs flow coefficient calculation processing (in Step S304). The flow rate measurer 206 calculates the flow coefficient $R_K$ by performing the flow coefficient calculation processing.

The flow rate measurer 206 calculates the flow rate Q using the provisional flow rate $Q_0$ and flow coefficient $R_K$ thus calculated and Mathematical Expression 14 (in Step S305).

The flow rate measurer 206 calculates, based on the flow rate Q thus calculated, a standard flow rate $Q_n$ of the gas mixtures which is converted into a value corresponding to the condition including 0°C and 1 atm (in Step S306).

The flow rate measurer 206 acquires the hydrogen concentration $x_1$ calculated in Step S274 and multiplies the standard flow rate Qn thus calculated by the hydrogen concentration $x_1$ thus acquired, thereby calculating the flow rate of hydrogen (in Step S307). Alternatively, the flow rate measurer 206 may also obtain the flow rate of hydrogen by multiplying the flow rate Q thus calculated by the hydrogen concentration $x_1$ as a gas concentration.

(3.10) Flow coefficient calculation processing

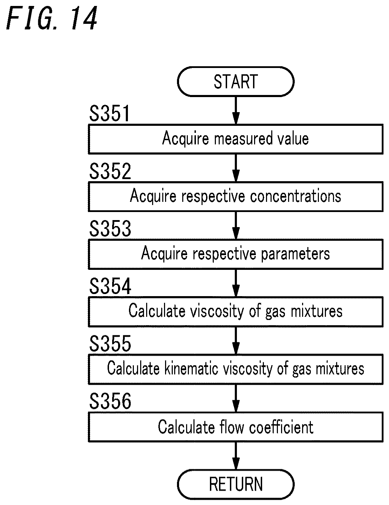

In this section, the flow coefficient calculation processing in Step S304 shown in FIG. 13 will be described with reference to FIG. 14.

The flow rate measurer 206 acquires the temperature T and the pressure $P_m$ (in Step S351). The temperature T is an average value of the first temperature value measured by the first temperature sensor 13 and the second temperature value measured by the second temperature sensor 14.

The flow rate measurer 206 acquires the respective concentrations of water vapor, hydrogen, and nitrogen included in the gas mixtures (in Step S352). That is to say, the flow rate measurer 206 acquires the hydrogen concentration $x_1$, the nitrogen concentration $x_2$, and the water vapor concentration $x_w$ ($= x_3$). In this case, the nitrogen concentration $x_2$ is given by $1 - x_1 - x_w$.

The flow rate measurer 206 acquires the respective parameters that have been stored in advance (in Step S353). Specifically, the flow rate measurer 206 acquires, as the respective parameters, the characteristic length D and the cross-sectional area S of the flow channel 101. In addition, the flow rate measurer 206 also acquires the viscosities of the respective gases (namely, hydrogen, nitrogen, and water vapor) included in the gas mixtures.

The flow rate measurer 206 calculates the viscosity $\mu$ of the gas mixtures including hydrogen, nitrogen, and water vapor using the respective viscosities of hydrogen, nitrogen, and water vapor, the water vapor concentration $x_w$, the hydrogen concentration $x_1$, and the nitrogen concentration $x_2$ (in Step S354).

The flow rate measurer 206 calculates the kinematic viscosity v of the gas mixtures including hydrogen, nitrogen, and water vapor using the water vapor concentration $x_w$, the hydrogen concentration $x_1$, the nitrogen concentration $x_2$. and Mathematical Expression 16 (in Step S355).

The flow rate measurer 206 calculates a flow coefficient $R_K$ corresponding to the provisional flow rate $Q_0$ by locating an intersection A0 between a graph represented by the relational expression obtained by advance evaluation and a graph expressed by Mathematical Expression (19) (in Step S356).

(3.11) Relative humidity measurement processing

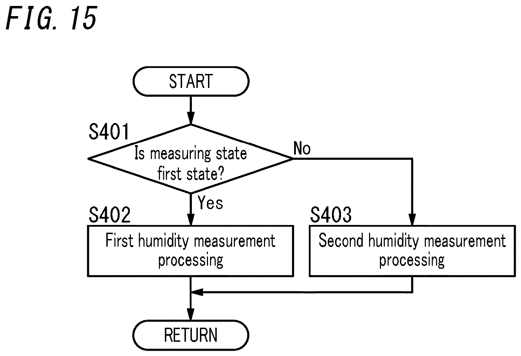

In this section, the relative humidity measurement processing in Step S9 shown in FIG. 5 will be described with reference to FIG. 15.

The relative humidity measurer 207 determines whether or not the measuring state is the first state (in Step S401).

When determining the measuring state to be the first state (if the answer is Yes in Step S401), the relative humidity measurer 207 performs first humidity measurement processing (in Step S402). Specifically, the relative humidity measurer 207 obtains the relative humidity of the gas mixtures using the water vapor pressure $P_W$ calculated by using Mathematical Expression 1, the saturated water vapor pressure $P_{WS}$ with respect to the temperature measured by the second temperature sensor 14, and the Mathematical Expression 20 described above.

When determining that the measuring state not be the first state, i.e., when determining the measuring state to be the second state (if the answer is No in Step S401), the relative humidity measurer 207 performs second humidity measurement processing (in Step S403). Specifically, the relative humidity measurer 207 obtains the relative humidity of the gas mixtures using the water vapor pressure $P_W$ calculated by using Mathematical Expression 2, the saturated water vapor pressure $P_{WS}$ with respect to the temperature measured by the second temperature sensor 14, and the Mathematical Expression 21 described above.

(4) Advantages

As can be seen from the foregoing description, a physical quantity measurement system 1 according to this embodiment includes a temperature and humidity sensor 16, a heat generator 17, a heat generation controller 203, and a water vapor pressure measurer 204. The temperature and humidity sensor 16 measures a humidity and temperature of gas mixtures flowing through a flow channel 101. The heat generator 17 performs a heat generating operation that generates heat to warm the temperature and humidity sensor 16. The heat generation controller 203 controls ON/OFF states of the heat generator 17 to cause the heat generator 17 to switch from an ON state where the heat generator 17 performs the heat generating operation to an OFF state where the heat generator 17 stops performing the heat generating operation, and vice versa. The water vapor pressure measurer 204 measures a water vapor pressure of water vapor included in the gas mixtures. The heat generation controller 203 controls the ON/OFF states of the heat generator 17 based on a result of comparison between a humidity measured value of the gas mixtures that has been measured by the temperature and humidity sensor 16 and a threshold value. The water vapor pressure measurer 204 measures, irrespective of the state of the heat generator 17, the water vapor pressure of the water vapor based on the humidity measured value and a temperature measured value of the gas mixtures that has been measured by the temperature and humidity sensor 16.

This configuration may reduce, by controlling the heat generation operation by the heat generator 17, the likelihood of producing condensation in the temperature and humidity sensor 16 even if the gas mixtures flow through the flow channel under a high-humidity environment. Consequently, the water vapor pressure of the gas mixtures may be measured more accurately even when the gas mixtures flow through the flow channel under the high-humidity environment.

(5) Variations

Next, variations will be enumerated one after another. Note that the variations to be described below may be adopted in combination as appropriate.

(5.1) First variation

In the exemplary embodiment described above, the processing device 20 is configured to change the measuring state using the first threshold value and the second threshold value, i.e., configured to change the measuring method using the first threshold value and the second threshold value. However, this configuration is only an example and should not be construed as limiting.

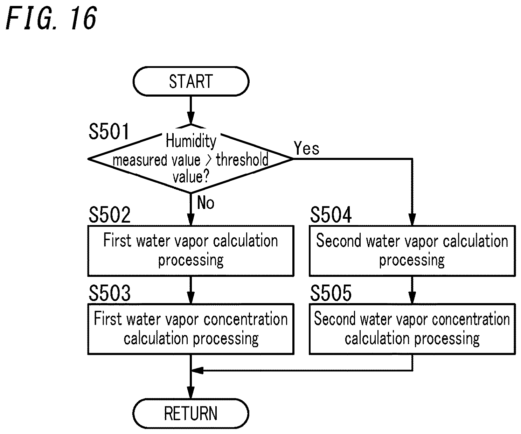

Alternatively, the processing device 20 may also change the measuring method using only one threshold value. The third measurement processing according to this first variation will now be described with reference to FIG. 16.

The water vapor pressure measurer 204 determines whether or not the humidity measured value obtained by the second signal processor 202 is greater than a predefined threshold value (in Step S501). For example, the predefined threshold value may be the second threshold value described above.

When determining that the humidity measured value not be greater than the predefined threshold value (second threshold value), i.e., when determining the humidity measured value to be equal to or less than the second threshold value (if the answer is No in Step S501), the water vapor pressure measurer 204 performs first water vapor pressure calculation processing (in Step S502). Specifically, the water vapor pressure measurer 204 calculates the water vapor pressure $P_W$ using the Mathematical Expression 1 described above. Thereafter, the concentration measurer 205 performs the first water vapor concentration calculation processing (in Step S503). The concentration measurer 205 measures the water vapor concentration $x_w$ of water vapor included in the gas mixtures using the water vapor pressure $P_W$ obtained in Step S502, the pressure $P_m$ of the gas mixtures that has been obtained by the second signal processor 202, and the Mathematical Expression 3 described above.

When determining the humidity measured value to be greater than the predefined threshold value (second threshold value) (if the answer is Yes in Step S501), the water vapor pressure measurer 204 performs second water vapor pressure calculation processing (in Step S504). Specifically, the water vapor pressure measurer 204 calculates the water vapor pressure $P_W$ using the Mathematical Expression 2 described above. Thereafter, the concentration measurer 205 performs the second water vapor concentration calculation processing (in Step S505). The concentration measurer 205 measures the water vapor concentration $x_w$ of water vapor included in the gas mixtures using the water vapor pressure $P_W$ obtained in Step S504, the pressure $P_m$ of the gas mixtures that has been obtained by the second signal processor 202, and the Mathematical Expression 3 described above.

Also, according to the first variation, if the processing device 20 performs the relative humidity measurement processing, the relative humidity measurer 207 determines, in Step S401, whether or not the humidity measured value is greater than a predefined threshold value, instead of determining whether or not the measuring state is the first state. When determining that the humidity measured value not be greater than the predefined threshold value (second threshold value), i.e., when determining the humidity measured value to be equal to or less than the second threshold value, the relative humidity measurer 207 performs first humidity measurement processing shown in Step S402. On the other hand, when determining the humidity measured value to be greater than the predefined threshold value (second threshold value), the relative humidity measurer 207 performs the second humidity measurement processing shown in Step S403.

Alternatively, when determining, in Step S501, the humidity measured value to be greater than the predefined threshold value (second threshold value), the water vapor pressure measurer 204 may set the measuring state at the second measuring state. On the other hand, when determining, in Step S501, that the humidity measured value not be greater than the predefined threshold value (second threshold value), the water vapor pressure measurer 204 may set the measuring state at the first measuring state. In that case, there is no need to change Step S401 of the relative humidity measurement processing as described above.

(5.2) Second variation

When detecting any abnormality in the heat generating operation, the control unit 25 of the processing device 20 may control the power feeding unit 26 to suspend supplying electric power to at least the heat generator 17 among the respective sensors and heat generator 17 provided for the flow channel 101.

(5.3) Third variation

In the exemplary embodiment described above, a so-called "Z-path" in which the pair of ultrasonic transducers 11, 12 are arranged to form a tilt angle $\theta$ with respect to the flow channel 101 is used as the ultrasonic wave propagation path 106. However, this configuration is only an example and should not be construed as limiting.

Alternatively, a path involving reflection, such as a so-called "V-path," i.e., a path formed by arranging the pair of ultrasonic transducers 11, 12 to cause an ultrasonic wave to travel across the flow of the gas mixtures twice in the flow channel 101, may also be adopted as the ultrasonic wave propagation path.

(5.4) Fourth variation

In the embodiment described above, the gas mixtures are supposed to include nitrogen as an additional component of the gas mixtures other than hydrogen. However, this configuration is only an example and should not be construed as limiting. Optionally, the gas mixtures may also include, as gases other than hydrogen, hydrocarbon (HC) such as methane, carbon dioxide, helium, argon, and oxygen, not just nitrogen.

(Other variations)

Note that the embodiment described above is only an exemplary one of various embodiments of the present disclosure and should not be construed as limiting. Rather, the exemplary embodiment may be readily modified in various manners depending on a design choice or any other factor without departing from the scope of the present disclosure.

The functions of the physical quantity measurement system 1 may also be implemented as, for example, a physical quantity measurement method, a computer program, or a non-transitory storage medium on which the program is stored. A physical quantity measurement method according to an aspect is designed for use in a physical quantity measurement system 1 including a temperature and humidity sensor 16 and a heat generator 17. The temperature and humidity sensor 16 measures a humidity and temperature of gas mixtures flowing through a flow channel 101. The heat generator 17 performs a heat generating operation that generates heat to warm the temperature and humidity sensor 16. The physical quantity measurement method includes a heat generation control step and a water vapor pressure measurement step. The heat generation control step includes controlling ON/OFF states of the heat generator 17 to cause the heat generator 17 to switch from an ON state where the heat generator 17 performs the heat generating operation to an OFF state where the heat generator 17 stops performing the heat generating operation, and vice versa. The water vapor pressure measurement step includes measuring a water vapor pressure of water vapor included in the gas mixtures. The heat generation control step includes controlling the ON/OFF states of the heat generator 17 based on a result of comparison between a humidity measured value of the gas that has been measured by the temperature and humidity sensor 16 and a threshold value. The water vapor pressure measurement step includes measuring, irrespective of the state of the heat generator 17, the water vapor pressure of the water vapor based on the humidity measured value and a temperature measured value of the gas mixtures that has been measured by the temperature and humidity sensor 16. A program according to another aspect is designed to cause a computer system to serve as an agent that performs the physical quantity measurement method described above.

The physical quantity measurement system 1 according to the present disclosure includes a computer system. The computer system includes a processor and a memory as principal hardware components thereof. The computer system performs the functions of the physical quantity measurement system 1 according to the present disclosure by making the processor execute a program stored in the memory of the computer system. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be made up of a single or a plurality of electronic circuits including a semiconductor integrated circuit (IC) or a large-scale integrated circuit (LSI). As used herein, the "integrated circuit" such as an IC or an LSI is called by a different name depending on the degree of integration thereof. Examples of the integrated circuits such as an IC or an LSI include integrated circuits called a "system LSI," a "very-large-scale integrated circuit (VLSI)," and an "ultra-large-scale integrated circuit (ULSI)." Optionally, a field-programmable gate array (FPGA) to be programmed after an LSI has been fabricated or a reconfigurable logic device allowing the connections or circuit sections inside of an LSI to be reconfigured may also be adopted as the processor. Those electronic circuits may be either integrated together on a single chip or distributed on multiple chips, whichever is appropriate. Those multiple chips may be aggregated together in a single device or distributed in multiple devices without limitation. As used herein, the "computer system" includes a microcontroller including one or more processors and one or more memories. Thus, the microcontroller may also be implemented as a single or a plurality of electronic circuits including a semiconductor integrated circuit or a large-scale integrated circuit.

In the embodiment described above, the plurality of functions of the physical quantity measurement system 1 are integrated together in a single housing. However, this is not an essential configuration for the physical quantity measurement system 1. Alternatively, those constituent elements of the physical quantity measurement system 1 may be distributed in multiple different housings. Still alternatively, at least some functions of the physical quantity measurement system 1 may be implemented as a cloud computing system as well.

(Recapitulation)

As can be seen from the foregoing description, a physical quantity measurement system (1) according to a first aspect includes a temperature and humidity sensor (16), a heat generator (17), a heat generation controller (203), and a water vapor pressure measurer (204). The temperature and humidity sensor (16) measures a humidity and temperature of gas mixtures flowing through a flow channel (101). The heat generator (17) performs a heat generating operation that generates heat to warm the temperature and humidity sensor (16). The heat generation controller (203) controls ON/OFF states of the heat generator (17) to cause the heat generator (17) to switch from an ON state where the heat generator (17) performs the heat generating operation to an OFF state where the heat generator (17) stops performing the heat generating operation, and vice versa. The water vapor pressure measurer (204) measures a water vapor pressure of water vapor included in the gas mixtures. The heat generation controller (203) controls the ON/OFF states of the heat generator (17) based on a result of comparison between a humidity measured value of the gas mixtures that has been measured by the temperature and humidity sensor (16) and a threshold value. The water vapor pressure measurer (204) measures, irrespective of the state of the heat generator (17), the water vapor pressure of the water vapor based on the humidity measured value and a temperature measured value of the gas mixtures that has been measured by the temperature and humidity sensor (16).

This aspect may reduce, by controlling the heat generation operation by the heat generator (17), the likelihood of producing condensation in the temperature and humidity sensor (16) even if the gas mixtures flow through the flow channel under a high-humidity environment. Consequently, the water vapor pressure of the gas mixtures may be measured more accurately even when the gas mixtures flow through the flow channel under the high-humidity environment.

A physical quantity measurement system (1) according to a second aspect, which may be implemented in conjunction with the first aspect, further includes a pressure sensor (15) and a concentration measurer (205). The pressure sensor (15) measures a pressure of the gas mixtures. The concentration measurer (205) measures a water vapor concentration of the water vapor included in the gas mixtures based on the water vapor pressure measured by the water vapor pressure measurer (204) and the pressure of the gas mixtures that has been measured by the pressure sensor (15).

According to this aspect, the water vapor concentration of the water vapor included in the gas mixtures is measured by using the water vapor pressure measured by the water vapor pressure measurer (204), thus allowing the water vapor concentration to be measured more accurately.

A physical quantity measurement system (1) according to a third aspect, which may be implemented in conjunction with the second aspect, further includes a pair of ultrasonic transducers (11, 12). The pair of ultrasonic transducers (11, 12) transmit and receive an ultrasonic wave. The pair of ultrasonic transducers (11, 12) are arranged to cause the ultrasonic wave to propagate across a flow of the gas mixtures through the flow channel (101). The concentration measurer (205) further measures a gas concentration as a concentration of a gas included in the gas mixtures and different from the water vapor based on the water vapor concentration and a propagation time of the ultrasonic wave between the pair of ultrasonic transducers (11, 12).

According to this aspect, a gas concentration that is the concentration of a gas included in the gas mixtures and different from the water vapor is measured by using the water vapor concentration measured by the concentration measurer (205), thus allowing the gas concentration to be measured more accurately.

A physical quantity measurement system (1) according to a fourth aspect, which may be implemented in conjunction with the third aspect, further includes a flow rate measurer (206). The flow rate measurer (206) measures a flow rate of the gas mixtures in the flow channel (101) using the propagation time of the ultrasonic wave, the water vapor concentration, and the gas concentrations and measures a flow rate of the gas using the flow rate of the gas mixtures.

According to this aspect, the flow rate of the gas mixtures is measured by using the water vapor concentration measured by the concentration measurer (205) and the gas concentration, thus allowing the flow rate of the gas mixtures to be measured more accurately.

A physical quantity measurement system (1) according to a fifth aspect, which may be implemented in conjunction with any one of the first to fourth aspects, further includes a temperature sensor (such as a second temperature sensor 14) and a relative humidity measurer

(207). The temperature sensor measures the temperature of the gas mixtures. The temperature sensor is disposed in a place where the temperature sensor is affected less significantly by the heat generated by the heat generator (17) than the temperature and humidity sensor (16) is. The relative humidity measurer (207) measures a relative humidity of the gas mixtures based on the water vapor pressure measured by the water vapor pressure measurer (204) and a saturated water vapor pressure corresponding to the temperature provided by the temperature sensor.

According to this aspect, the temperature sensor is affected less significantly by the heat generated by the heat generator (17) than the temperature and humidity sensor (16) is, and therefore, may measure the temperature in the flow channel (101) more accurately than the temperature measured by the temperature and humidity sensor (16). In addition, when the heat generator (17) is in ON state, the temperature and humidity sensor (16) is affected by the heat generated by the heat generator (17), and therefore, cannot measure the temperature in the flow channel (10) accurately. Thus, the temperature in the flow channel (101) may be measured more accurately by using the temperature measured by the temperature sensor even when the heat generator (17) is in ON state. Consequently, the relative humidity may be measured more accurately.

In a physical quantity measurement system (1) according to a sixth aspect, which may be implemented in conjunction with any one of the first to fifth aspects, the threshold value is a humidity value at which no condensation is produced in the temperature and humidity sensor (16).

According to this aspect, the likelihood of producing condensation in the temperature and humidity sensor (16) may be reduced by setting the threshold value at such a humidity value at which no condensation is produced. This may reduce the likelihood of causing a decline in measurement accuracy due to the production of condensation in the temperature and humidity sensor (16), thus allowing the water vapor pressure in the gas mixtures to be measured more accurately.

In a physical quantity measurement system (1) according to a seventh aspect, which may be implemented in conjunction with any one of the first to sixth aspects, when the humidity measured value is greater than the threshold value, the heat generation controller (203) controls, by pulse width modulation, a period in which the heat generator (17) turns ON and a period in which the heat generator (17) turns OFF, thereby alternately turning the heat generator (17) ON and OFF. The heat generation controller (203) controls a pulse width of the pulse width modulation such that the greater a difference between the threshold value and the humidity measured value is, the longer the ON state lasts. When the humidity measured value is equal to or less than the threshold value, the heat generation controller (203) turns the heat generator (17) OFF.

This aspect may reduce the likelihood of producing condensation in the temperature and humidity sensor (16).

**Reference Signs List**

1       Physical Quantity Measurement System
11     Ultrasonic Transducer (First Ultrasonic Transducer)
12     Ultrasonic Transducer (Second Ultrasonic Transducer)

| | |
|---|---|
| 13 | First Temperature Sensor |
| 14 | Second Temperature Sensor (Temperature Sensor) |
| 15 | Pressure Sensor |
| 16 | Temperature and Humidity Sensor |
| 17 | Heat Generator |
| 101 | Flow Channel |
| 203 | Heat Generation Controller |
| 204 | Water Vapor Pressure Measurer |
| 205 | Concentration Measurer |
| 206 | Flow Rate Measurer |
| 207 | Relative Humidity Measurer |

**Claims**

1.  A physical quantity measurement system comprising:

    a temperature and humidity sensor configured to measure a humidity and temperature of gas mixtures flowing through a flow channel;
    a heat generator configured to perform a heat generating operation that generates heat to warm the temperature and humidity sensor;
    a heat generation controller configured to control ON/OFF states of the heat generator to cause the heat generator to switch from an ON state where the heat generator performs the heat generating operation to an OFF state where the heat generator stops performing the heat generating operation, and vice versa; and
    a water vapor pressure measurer configured to measure a water vapor pressure of water vapor included in the gas mixtures,
    the heat generation controller being configured to control the ON/OFF states of the heat generator based on a result of comparison between a humidity measured value of the gas mixtures that has been measured by the temperature and humidity sensor and a threshold value, and
    the water vapor pressure measurer being configured to measure, irrespective of a state of the heat generator, the water vapor pressure of the water vapor included in the gas mixtures based on the humidity measured value and a temperature measured value of the gas mixtures that has been measured by the temperature and humidity sensor.

2.  The physical quantity measurement system of claim 1, further comprising:

    a pressure sensor configured to measure a pressure of the gas mixtures; and
    a concentration measurer configured to measure a water vapor concentration of the water vapor included in the gas mixtures based on the water vapor pressure measured by the water vapor pressure measurer and the pressure of the gas mixtures that has been measured by the pressure sensor.

3.  The physical quantity measurement system of claim 2, further comprising

    a pair of ultrasonic transducers arranged to transmit and receive an ultrasonic wave and cause the ultrasonic wave to propagate across a flow of the gas mixtures through the flow channel, wherein
    the concentration measurer is configured to further measure a gas concentration as a concentration of a gas included in the gas mixtures and different from the water vapor based on the water vapor concentration and a propagation time of the ultrasonic wave between the pair of ultrasonic transducers.

4.  The physical quantity measurement system of claim 3, further comprising
    a flow rate measurer configured to measure a flow rate of the gas mixtures in the flow channel using the propagation time of the ultrasonic wave, the water vapor concentration, and the gas concentrations and measure a flow rate of the gas using the flow rate of the gas mixtures.

5.  The physical quantity measurement system of any one of claims 1 to 4, further comprising:

    a temperature sensor configured to measure the temperature of the gas mixtures, the temperature sensor being disposed in a place where the temperature sensor is affected less significantly by the heat generated by the heat generator than the temperature and humidity sensor is; and

a relative humidity measurer configured to measure a relative humidity of the gas mixtures based on the water vapor pressure measured by the water vapor pressure measurer and a saturated water vapor pressure corresponding to the temperature provided by the temperature sensor.

6. The physical quantity measurement system of any one of claims 1 to 5, wherein
   the threshold value is a humidity value at which no condensation is produced in the temperature and humidity sensor.

7. The physical quantity measurement system of any one of claims 1 to 6, wherein
   the heat generation controller is configured to:

   when the humidity measured value is greater than the threshold value, control, by pulse width modulation, a period in which the heat generator turns ON and a period in which the heat generator turns OFF, thereby alternately turning the heat generator ON and OFF;
   control a pulse width of the pulse width modulation such that the greater a difference between the threshold value and the humidity measured value is, the longer the ON state lasts; and
   when the humidity measured value is equal to or less than the threshold value, turn the heat generator OFF.

## FIG. 1

FIG. 2

FIG. 3

FIG. 4

*FIG. 5*

START

S1
First measurement processing

S2
Second measurement processing

S3
Heat generation control processing

S4
Third measurement processing

S5
First abnormality detection processing

S6
Second abnormality detection processing

S7
Concentration measurement processing

S8
Flow rate measurement processing

S9
Relative humidity measurement processing

END

*FIG. 6*

```
          ┌──────────────┐
          │    START     │
          └──────────────┘
                 │
 S51             ▼
┌──────────────────────────────────┐
│ Acquire humidity measured value  │
└──────────────────────────────────┘
                 │
 S52             ▼
          ╱──────────────╲
         ╱    Humidity     ╲          No
        ⟨ measured value ⟩ first ⟩──────────────┐
         ╲  threshold value? ╱                  │
          ╲──────────────╱                      │
                 │ Yes                          │
 S53             ▼                   S54         ▼
┌─────────────────────────┐   ┌─────────────────────────┐
│ Set operating state of  │   │ Set operating state of  │
│  heat generator at ON   │   │  heat generator at OFF  │
│         state           │   │         state           │
└─────────────────────────┘   └─────────────────────────┘
                 │                           │
                 ▼◄──────────────────────────┘
          ┌──────────────┐
          │   RETURN     │
          └──────────────┘
```

*FIG. 7*

```
                    ┌──────────────┐
                    │    START     │
                    └──────┬───────┘
                           │
      S101          ╱──────▼──────╲           No
                   ╱  Is measuring  ╲ ─────────────────────────┐
                   ╲ state first state? ╱                       │
                    ╲──────┬──────╱                             │
                           │ Yes                                │
      S102          ╱──────▼──────╲     Yes       S106   ╱──────▼──────╲      No
                   ╱   Humidity     ╲──────┐            ╱   Humidity     ╲──────┐
                   ╲ measured value >  ╱    │            ╲ measured value >  ╱    │
                   ╲  first         ╱    │            ╲  second        ╱    │
                   ╲ threshold value? ╱   │            ╲ threshold value? ╱   │
                    ╲──────┬──────╱       │             ╲──────┬──────╱       │
                           │ No          (a)                   │ Yes         (b)
                       ┌───◄──(b)                          ┌───◄──(a)
      S103             │                     S107          │
              ┌────────▼────────┐                  ┌───────▼─────────┐
              │ Set measuring    │                  │ Set measuring    │
              │ state at         │                  │ state at         │
              │ first state      │                  │ second state     │
              └────────┬────────┘                  └───────┬─────────┘
      S104             │                     S108          │
              ┌────────▼────────┐                  ┌───────▼─────────┐
              │ First water vapor│                  │ Second water vapor│
              │ pressure          │                  │ pressure          │
              │ calculation       │                  │ calculation       │
              │ processing        │                  │ processing        │
              └────────┬────────┘                  └───────┬─────────┘
      S105             │                     S109          │
              ┌────────▼────────┐                  ┌───────▼─────────┐
              │ First water vapor│                  │ Second water vapor│
              │ concentration     │                  │ concentration     │
              │ calculation       │                  │ calculation       │
              │ processing        │                  │ processing        │
              └────────┬────────┘                  └───────┬─────────┘
                       │◄──────────────────────────────────┘
                  ┌────▼─────┐
                  │  RETURN  │
                  └──────────┘
```

*FIG. 8*

```
                    ( START )
S151                    │
┌──────────────────────▼──────────────────────┐
│  Obtain standard deviation of humidity       │
│             measured value                   │
└──────────────────────┬──────────────────────┘
S152                    │
┌──────────────────────▼──────────────────────┐
│        Determine reference value             │
│ corresponding to water vapor pressure        │
└──────────────────────┬──────────────────────┘
                        │
S153                    ▼
        ╱────────────────────────────╲              No
       ⟨   Standard deviation          ⟩──────────────┐
        ╲  ⟩ reference value?         ╱               │
         ╲────────────────────────────╱               │
                        │ Yes                          │
S154                    ▼                              │
┌──────────────────────────────────────────────┐      │
│       Detect abnormality in measuring         │      │
│              environment                      │      │
└──────────────────────┬───────────────────────┘      │
S155                    │                              │
┌──────────────────────▼───────────────────────┐      │
│        Output first detection result          │      │
└──────────────────────┬───────────────────────┘      │
                        │◄─────────────────────────────┘
                        ▼
                   ( RETURN )
```

*FIG. 9*

```
              ┌──────────────┐
              │    START     │
              └──────┬───────┘
                     │
                     ▼
S201   ╱────────────────────────╲        No
      ╱  First differential value >╲ ──────────┐
      ╲  first comparison value?    ╱          │
       ╲──────────┬────────────────╱           │
                  │ Yes                         │
                  ▼                             │
S202   ╱────────────────────────╲        No     │
      ╱  Second differential value >╲ ──────────┤
      ╲  second comparison value?   ╱           │
       ╲──────────┬────────────────╱            │
                  │ Yes                          │
                  ▼                              │
S203   ╱────────────────────────╲        No      │
      ╱  Temperature measured value╲ ──────────┤
      ╲  > third comparison value? ╱            │
       ╲──────────┬────────────────╱            │
                  │ Yes                          │
S204              ▼                              │
      ┌────────────────────────────────────┐    │
      │ Detect abnormality in heat generating operation │
      └──────────────┬─────────────────────┘    │
S205                 ▼                           │
      ┌────────────────────────────────────┐    │
      │    Output second result of detection │    │
      └──────────────┬─────────────────────┘    │
S206                 ▼                           │
      ┌────────────────────────────────────┐    │
      │         Cutoff processing           │    │
      └──────────────┬─────────────────────┘    │
                     │◄──────────────────────────┘
                     ▼
              ┌──────────────┐
              │    RETURN     │
              └──────────────┘
```

*FIG. 10*

```
        ┌─────────────┐
        (    START    )
S251          │
 ┌────────────▼─────────────────┐
 │ Speed of sound calculation processing │
 └──────────────────────────────┘
S252          │
 ┌────────────▼─────────────────┐
 │ Hydrogen concentration calculation processing │
 └──────────────────────────────┘
              │
        (   RETURN    )
        └─────────────┘
```

*FIG. 11*

```
        ( START )
S261        │
┌──────────────────────────────────────┐
│ Acquire distance between ultrasonic    │
│ sensors, first propagation time, and   │
│ second propagation time                │
└──────────────────────────────────────┘
S262        │
┌──────────────────────────────────────┐
│   Calculate average propagation time   │
└──────────────────────────────────────┘
S263        │
┌──────────────────────────────────────┐
│       Calculate speed of sound         │
└──────────────────────────────────────┘
            │
        ( RETURN )
```

*FIG. 12*

```
         ┌─────────────┐
         │    START    │
         └──────┬──────┘
S271            │
┌───────────────▼──────────────┐
│Acquire temperature measured value│
└──────────────────────────────┘
S272            │
┌───────────────▼──────────────┐
│   Acquire speed of sound and  │
│    water vapor concentration  │
└──────────────────────────────┘
S273            │
┌───────────────▼──────────────┐
│  Acquire respective parameters │
└──────────────────────────────┘
S274            │
┌───────────────▼──────────────┐
│ Calculate hydrogen concentration│
└──────────────────────────────┘
                │
         ┌──────▼──────┐
         │   RETURN    │
         └─────────────┘
```

# FIG. 13

```
                      START
S301
      Acquire first propagation time and
            second propagation time
S302
         Acquire respective parameters
S303
     Provisional flow rate calculation processing
S304
      Flow coefficient calculation processing
S305
        Calculate flow rate of gas mixtures
S306
           Calculate standard flow rate
S307
         Calculate flow rate of hydrogen

                      RETURN
```

## FIG. 14

```
                    ( START )
 S351                   │
        ┌───────────────▼───────────────┐
        │     Acquire measured value     │
 S352   └───────────────┬───────────────┘
        ┌───────────────▼───────────────┐
        │  Acquire respective concentrations  │
 S353   └───────────────┬───────────────┘
        ┌───────────────▼───────────────┐
        │   Acquire respective parameters    │
 S354   └───────────────┬───────────────┘
        ┌───────────────▼───────────────┐
        │  Calculate viscosity of gas mixtures  │
 S355   └───────────────┬───────────────┘
        ┌───────────────▼───────────────┐
        │ Calculate kinematic viscosity of gas mixtures │
 S356   └───────────────┬───────────────┘
        ┌───────────────▼───────────────┐
        │      Calculate flow coefficient     │
        └───────────────┬───────────────┘
                        │
                    ( RETURN )
```

*FIG. 15*

```
                    ┌──────────────┐
                    │    START     │
                    └──────┬───────┘
                           │
    S401                   ▼
        ┌──────────────────────────────────┐  No
        │      Is measuring state           ├──────────────┐
        │        first state?               │              │
        └──────────────┬───────────────────┘              │
                       │ Yes                               │
    S402               ▼                 S403              ▼
    ┌────────────────────────┐      ┌─────────────────────────┐
    │ First humidity measurement│    │ Second humidity measurement│
    │      processing          │    │       processing          │
    └────────────┬───────────┘      └────────────┬────────────┘
                 │                                │
                 ▼◄───────────────────────────────┘
         ┌──────────────┐
         │   RETURN     │
         └──────────────┘
```

## FIG. 16

```
                    ┌─────────────┐
                    │    START    │
                    └──────┬──────┘
                           │
    S501              ╱────┴────╲
               ╱──── Humidity ────╲        Yes
              ╲  measured value ⟩ threshold ╲──────────┐
               ╲──── value? ────╱                      │
    S502           │ No                    S504        │
    ┌──────────────┴──────────┐         ┌──────────────┴──────────┐
    │ First water vapor        │         │ Second water vapor       │
    │ calculation processing   │         │ calculation processing   │
    └──────────────┬──────────┘         └──────────────┬──────────┘
    S503           │                     S505           │
    ┌──────────────┴──────────┐         ┌──────────────┴──────────┐
    │ First water vapor        │         │ Second water vapor       │
    │ concentration            │         │ concentration            │
    │ calculation processing   │         │ calculation processing   │
    └──────────────┬──────────┘         └──────────────┬──────────┘
                   │◄──────────────────────────────────┘
                   │
            ┌──────┴──────┐
            │   RETURN    │
            └─────────────┘
```

41

## INTERNATIONAL SEARCH REPORT

| | International application No. |
|---|---|
| | **PCT/JP2024/003808** |

**A. CLASSIFICATION OF SUBJECT MATTER**

*G01N 25/56*(2006.01)i; *G01N 29/024*(2006.01)i
FI:   G01N25/56 B; G01N29/024

According to International Patent Classification (IPC) or to both national classification and IPC

**B. FIELDS SEARCHED**

Minimum documentation searched (classification system followed by classification symbols)

G01N25/56; G01N29/024

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | CN 114839106 A (BEIJING SINOHYTEC CO., LTD.) 02 August 2022 (2022-08-02)<br>paragraphs [0057]-[0062] | 1-7 |
| A | JP 2018-119508 A (HONDA MOTOR CO., LTD.) 02 August 2018 (2018-08-02)<br>paragraphs [0031]-[0033] | 1-7 |
| A | JP 2022-025751 A (PANASONIC INTELLECTUAL PROPERTY MANAGEMENT CO.,<br>LTD.) 10 February 2022 (2022-02-10)<br>paragraphs [0018]-[0037] | 1-7 |
| P, A | WO 2023/238875 A1 (PANASONIC INTELLECTUAL PROPERTY MANAGEMENT CO.,<br>LTD.) 14 December 2023 (2023-12-14)<br>paragraph [0014] | 1-7 |

☐ Further documents are listed in the continuation of Box C.      ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **11 March 2024** | **26 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/003808**

| Patent document cited in search report | | | Publication date (day/month/year) | Patent family member(s) | | | Publication date (day/month/year) |
|---|---|---|---|---|---|---|---|
| CN | 114839106 | A | 02 August 2022 | (Family: none) | | | |
| JP | 2018-119508 | A | 02 August 2018 | (Family: none) | | | |
| JP | 2022-025751 | A | 10 February 2022 | US | 2023/0296568 | A1 | |
| | | | | paragraphs [0021]-[0040] | | | |
| | | | | WO | 2022/024821 | A1 | |
| | | | | EP | 4191208 | A1 | |
| | | | | CN | 116157656 | A | |
| WO | 2023/238875 | A1 | 14 December 2023 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003317752 A